(19) Europäisches Patentamt
European Patent Office
Office européen des brevets

(11) **EP 2 875 724 B1**

(12) **EUROPEAN PATENT SPECIFICATION**

(45) Date of publication and mention
of the grant of the patent:
**06.01.2016 Bulletin 2016/01**

(51) Int Cl.:
***A01G 33/00*** *(2006.01)*

(21) Application number: **13382470.6**

(22) Date of filing: **25.11.2013**

(54) **Open reactor for cultivating microalgae**

bassin ouvert pour la culture d algues

Offene systeme für die Algen Züchtung

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**27.05.2015 Bulletin 2015/22**

(73) Proprietor: **FCC Aqualia, S.A.
28016 Madrid (ES)**

(72) Inventors:
• **Lara Corona, Enrique
28050 Madrid (ES)**
• **Rogalla, Frank
28050 Madrid (ES)**

(74) Representative: **Ungria López, Javier
Avda. Ramón y Cajal, 78
28043 Madrid (ES)**

(56) References cited:
**US-A1- 2013 269 244**

• **KURT LIFFMAN ET AL: "Comparing the energy
efficiency of different high rate algal raceway
pond designs using computational fluid
dynamics", CHEMICAL ENGINEERING
RESEARCH AND DESIGN, vol. 91, no. 2, 1
February 2013 (2013-02-01), pages 221-226,
XP055113020, ISSN: 0263-8762, DOI:
10.1016/j.cherd.2012.08.007**
• **HARGREAVES J A: "Pond Mixing", INTERNET
CITATION, July 2003 (2003-07), pages 1-6,
XP002711247, Retrieved from the Internet: URL:
http://ces3.ca.uky.edu/westkentuckyaqu
aculture/Data/Aeration,%20Circulation%20an
d%20Water%20Destratification/
SRAC%204602%2 0Pond%20Mixing.pdf
[retrieved on 2013-08-13]**
• **PARK J B K ET AL: "Wastewater treatment high
rate algal ponds for biofuel production",
BIORESOURCE TECHNOLOGY, ELSEVIER BV,
GB, vol. 102, no. 1, 1 January 2011 (2011-01-01),
pages 35-42, XP027368455, ISSN: 0960-8524
[retrieved on 2010-07-31]**

**Description**

<u>**Technical Field**</u>

The present invention is encompassed within the field of the production of microalgae or cultivation of mixotrophic algae-bacteria, for the subsequent use thereof such as in liquid effluent treatment processes by means of their cultivation. Specifically, the invention is encompassed within the area of cultivation in open raceway or high rate algae pond (HRAP) reactor systems.

**State of the art**

Microalgae are highly useful microorganisms because they present beneficial applications in very different areas, such as the treatment of wastewater, the production of biofuels, bio-fertilizers, human and animal food, the reduction of greenhouse effect gases ($CO_2$), or the attainment of highly valuable chemical products. Microalgae cultivation can achieve levels of productivity that are much higher than the crops of superior plants, resulting in greater levels of $CO_2$ fixation and a greater amount of produced biomass. In addition, microalgae cultivation has fewer water needs and do not compete with traditional crops, given that they do not need fertile soil and can use water with low quality levels, such as wastewater and brine.

In the case of the treatment of wastewater, microalgae cultivation may have a fundamental role in the future. Cultures of mixotrophic algae-bacteria allow reducing the consumption of energy of traditional activated sludge systems, between 0.2 and 0.4 kw/m$^3$, given that the microalgae produce the oxygen necessary for bacteria to degrade organic matter and carry out the nitrification process. In addition, microalgae incorporate the N and P from wastewater during their photosynthetic growth, transforming it into biomass that can be recovered during the harvesting process, and thus preventing the dumping thereof and the environmental problems derived from said dumping, such as the eutrophication of receiving streams. In addition, they provide the highest level of pathogens disposal of all systems used for the biological treatment of wastewater.

There are mainly two differentiated classes within microalgae production systems: closed systems and open systems. Closed systems are characterized in that they isolate the fluid from the external environment and are less exposed to the perturbations thereof, whereas open systems are characterized in that they have a greater interaction or exposure to the environment and depend, to a greater extent, on the conditions thereof. Open systems (Oswald and Golueke, 1960) have had greater success at an industrial level because of their lower cost, although their use has been limited to certain extremophile species due to pollution problems. However, within the application of wastewater treatment, the control of cultured species is not as decisive, due to which these types of reactors have been the most widespread. These open reactors are known as raceway or HRAP (high rate algae pond) reactors. Even though the use of this type of reactors in wastewater treatment with microalgae has been described, they have not been appropriately optimized, which is being proposed as an essential challenge.

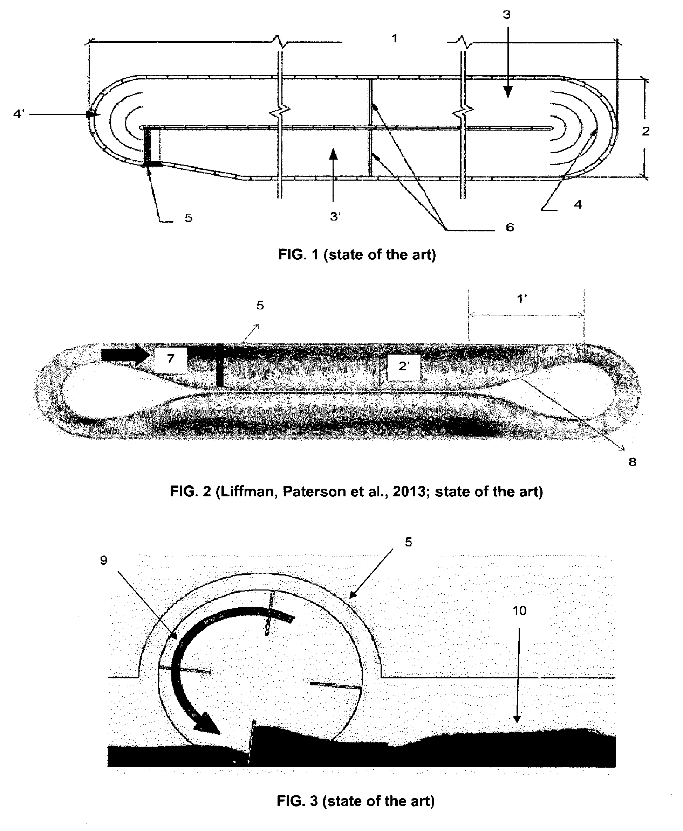

These open reactors, hereinafter referred to as HRAP, are open shallow ponds with a water sheet level ranging between 0.2 and 0.6 m, in the shape of a carrousel around an intermediate wall (two straight segments separated by a partition and joined to one another at their ends by means of curves). In these ponds, water is circulated at average horizontal speeds ranging between 0.2 and 0.4 m/s with the assistance of an agitation system or flow accelerator of the paddlewheel-type. Ponds can be built with a waterproofing sheet or without it, depending on the conditions of the soil and local regulations. $CO_2$ is usually added to the pond by means of a countercurrent injection system located in a pit, approximately 1.5 m deep, with a deflector. The curves are built with the same width than the straight segments of the channel or slightly narrower at the paddlewheel area, and with several curved deflectors to avoid the detachment of the flow and the formation of dead regions (Figure 1, state of the art).

The design of HRAP for the cultivation of algae has not been modified and has barely been improved since the 50's. In addition, they have two main problems, which reduce their efficiency and that are solved by means of the present invention:

    1) high amount of power consumed during the agitation process
    2) low productivity due to the settling of biomass.

With respect to the high amount of power consumed during the agitation of the culture, the same is mainly caused due to:

- hydraulic problems in the carbonation system
- hydraulic problems at the curves
- low hydraulic performance in the agitation system used.

Hydraulic problems in the carbonation system

The carbonation system used usually consists of a pit with a vertical deflector, where the gas with $CO_2$ is bubbled from the bottom, against the direction of the flow. This type of reflectors represent an abrupt obstacle for the horizontal flow in the HRAP reactor, causing an important load loss, which is the main loss in a conventional HRAP, and is estimated in values of the localized loss coefficient K (see below) close to 4. The configuration of carbonation systems has been recently improved by means of the elimination of the partition wall and by relocating the gas injection device, successfully enough so as to reduce these losses significantly (Spanish patent application P201231485).

Hydraulic problems at the curves

The 180° curves commonly used, such as those shown in Figure 1 (state of the art), have an average radius/curved channel width = 0.5, which is very small, that causes important load losses. The load losses h can be estimated from the localized loss coefficient K according to the following equation:

$$h = K \times \frac{v^2}{2g}$$

The K coefficient can have very high values in conventional curves. For the reduction of these losses, the most widely used designs to date (Sompech, Chisti et al. 2012) include the division of the curve into several channels by means of circular deflectors to minimize the areas of flow separation (see Figure 1, state of the art). These deflectors reduce the dead regions and the related load losses; however, when dividing the curve into as many as four channels, they cause load losses due to friction with K values of approximately 2, as reflected in Lundquist T.J. (2010). This value continues to be very high and must be minimized so these systems become more profitable. Another recent attempt at reducing the energy consumption at the curves appears in an article by Liffman, Paterson et al. (2013), who carried out a fluid-dynamic simulation by means of CFD techniques with the program *Ansys Fluent,* evidencing the possibility of the energy reduction at conventional curves in up to 87% thanks to a design with a variable depth and a constant section, with a narrowing from the straight segment of the channel to the drop-shaped curve (Figure 2, state of the art). However, this design has several problems:

- the narrowing is carried out asymmetrically with respect to the central axis of the straight channel, due to which the flow F is decentered when it exits the curve with respect to said axis. The foregoing causes a non-uniform distribution of speeds, with greater speeds in the external face of the straight channel. Likewise, this system requires conventional paddlewheel agitation systems, since it is hardly adaptable to submersible agitators.
- the expensive construction of the lateral walls and the bottom of the channel of the transitions due to the complexity of the cos(X) functions, necessary for a smooth transition.
- it does not include any carbonation or settleable solid elimination system.

Low hydraulic performance in the agitation system

The agitation system commonly used is, for example, the one referred to in the US patents 3,885,370 and 8,142,167. This system consists of a paddlewheel that propels the water when rotating around its axis, as described in Figure 3 (state of the art). The advantage of this system is that it allows working with low water sheet levels, just as those commonly used in the HARP, between 0.2 and 0.6 m. The problem is that this device has a very low hydraulic and global (hydraulic and mechanical) efficiency.

- Hydraulic performance = hydraulic agitation energy/energy at the rotational axis.
- Global energetic performance of the system = hydraulic agitation energy/electricity consumed.

Hydraulic performance values as low as 17% (Borowitzka, 2005) and 10% (Chiaramonti, Prussi et al, 2013), and global energetic performance values of the system as low as 4% (Richmond, 2003) or 5% (Chiaramonti, Prussi et al., 2013) have been reported, although some studies refer to higher values of approximately 40% (Weissan JC, 1989). The reason is that the paddles lose a significant part of the energy when pushing the flow downwards during the entry of the paddle into the water, and in pumping and extracting the water in a vertical manner during the exit of the paddle thereof.

There have been some attempts to replace the paddlewheel for other, more energy-efficient, devices. Specifically, high-speed submersible agitators or axial pumps (axial pumps are actually high-speed submersible agitators with a guided flow) have been used. For example, these high-speed agitators were used in the HARP for the cultivation of microalgae in the wastewater treatment plant in Riudecanyes, Tarragona (1,500 inhabitants; Garcia, J., and Mujeriego, R. (1999)), or in a recent study undertaken by Chiaramonti, Prussi et al. (2013). In this article, submersible agitators have high rotational speeds (normally > 700 rpm) and a small diameter (normally between 100 and 400 mm), and are placed in a narrowing of the main channel in the form of a *venturi*. By means of this high-speed agitator system, in the proposed reference, energy savings amounting to 60.5% in a 500 m²-pond, and 65% in a 5-m² pond, with respect to the conventional paddlewheel system, are claimed. However, this system has certain important problems:

a) Low hydraulic performance: the energetic performance associated with hydraulics in a submersible agitator depends on the power factor $e_z$ (N/kw), or the quotient between the thrust of $F_{propeller}$ (Newton) and the absorbed power $P_1$ (kw) at the working point.

$$e_z = \frac{F_{propeller}}{P_1}$$

The greater the power factor, the greater the efficiency of the agitator, given that it is able to propel a larger stream with less consumed energy. The power factor of high-speed submersible agitators with small diameters is a low value, comprised between 100 and 300 N/kw, which leads to low energetic performances.

b) High load losses in the *venturi* system: submersible agitators with high rotational speeds also have a high flow speed, often above 10 m/s. These flow speeds generate a contraction and an expansion from that speed up to reaching 0.2-0.3 m/s in the straight channel, and therefore, associated turbulences and important load losses.

c) Cellular stress: high rotational speeds above 100 rpm and normally above 700 rpm can affect some types of algae, especially filamentous and flagellated algae, although they prove in their article that there were no damages caused to the specific cultivation they were developing.

d) It needs a high submergence or water sheet over the propeller in order to avoid the vortex effect and cavitation due to the suction generated by the flow speed at the pump.

e) Low volumetric performance (measured as the ratio between the real effective flow rate at the HRAP and the flow rate driven by the propeller). The agitator of the aforementioned article is placed in an open channel *venturi* system, which causes backflow from the highest propulsion pressure to the lowest aspiration pressure through the clearance gaps between the propeller and the channel. These backflows cause the decrease of volumetric performance, in such a way that the real effective flow rate at the HRAP is lower than the effective flow rate driven by the propeller, sometimes as far as 50% less in some fluid-dynamic simulations carried out for this patent. Another solution to avoid these backflows is to place a wall to house the agitator, using one in this case in the form of an axial pump, as with the Riudecanyes (Tarragona) treatment plant. The problem is represented by the high load losses caused at the narrowing of the resulting hole and quite defective hydraulics, with dead regions. In fact, in this treatment plant, there were significant solid settling areas that prevented the correct operation of the system.

In terms of the low productivity caused by the settling of biomass, this is another of the usual problems found in HRAP, due to the uncontrolled accumulation of biomass in dead regions as a consequence of the defects in the hydraulic design commented above, mainly at the curves and at the bottom. These dead regions take place at conventional curves in the external faces of the deflectors and in the central dividing wall of the HRAP at the exit of the curve. This accumulation of biomass reduces the productivity of the algae, and therefore, the value associated with them. In addition, an uncontrolled settling process takes place at the bottom due to the defective agitation of the conventional paddlewheel system. Therefore, complex systems have been developed to extract the large amounts of biomass that this system is not able to keep suspended, such as the one presented in the US Patent 3,969,249. This high level of settling also took place at the treatment plant in Riudecanyes, where high-speed agitators with defective hydraulics were used.

In view of the energetic efficiency and productivity problems detected in open raceway or high rate algae pond reactor systems, even when the hydraulic design of the channel is improved (mainly at the curves thereof), the object of the present invention is to improve the design of agitation devices that are commonly used, in order to improve the hydrodynamics of the whole system in a joint and synergic manner.

**Bibliographic references**

Borowitzka, M. (2005). "Culturing microalgae in outdoor ponds." Algal Culturing Techniques. Andersen RA (Ed). Elsevier Academic Press, CA, USA: 204-218.

Chiaramonti, D., M. Prussi, et al. (2013). "Review of energy balance in raceway ponds for microalgae cultivation: Re-thinking a traditional system is possible." Applied Energy 102(0): 101-111.

Hager, W. H. (2010). "Wastewater Hydraulics, Theory and practice." (chapter 2): 33.

García, J., y Mujeriego, R. (1999). Oxigenacion fotosintética en lagunas para la depuración de aguas residuales. Tecnología del Agua 195, 57-64.

Liffman, K., D. A. Paterson, et al. (2013). "Comparing the energy efficiency of different high rate algal raceway pond designs using computational fluid dynamics." Chemical Engineering Research and Design 91(2): 221-226.

Lundquist TJ, W. I., Quinn NWT, Benemann JR (2010). "A realistic technology and engineering assessment of algae biofuel production." Energy Biosciences Institute.

Richmond, A. (2003). "Handbook of microalgal mass culture. Blackwell science Ltd,"

Sompech, K., Y. Chisti, et al. (2012). "Design of raceway ponds for producing microalgae." Biofuels 3(4): 387-397.

Weissman JC, T. D., Goebel RP (1989). "Design and operation of an outdoor microalgae test facility, Final Subcontract Report." Microbial Products Inc. Vacafille, California 95688.

Spanish patent application P201231485: Carbonation system for microalgae cultures in open reactors.

US 8,142,167: Paddlewheel apparatus

EP 2524962 A1: Algae Culture System

MX 201201334A: Microalgae growth pond design

US 3,855,370: Mixer for algae ponds

US 3,969,249: Solid Remover for High Rate Algae Ponds

## Brief description of the invention

The present invention relates to an open raceway or high rate algae pond (HRAP) reactor for the cultivation of microalgae or mixotrophic microalgae-bacteria cultures, wherein the consumption of energy of the agitation device is reduced between 80 and 90% with respect to conventional systems that are currently used, and taken into account that the productivity of algal biomass is also optimized thanks to the configuration of the main segments that make up the channel. The reduction in the consumption of energy has been carried out through a new HRAP design with hydraulic improvements and improvements in the agitation system. Accordingly, the present invention provides a high rate algae pond (HRAP) reactor as described in claim 1.

The open HRAP reactor object of the patent presents a flow channel through which the sheet of culture water with a water level ranging between 0.2 and 0.6m passes in the shape of a rounded rectangle with four sections: two straight parallel segments, separated by an intermediate partition and joined at their ends with (two) 180° curves in the shape of semicircles, each one of them provided with an entry and an exit depending on the direction in which the water sheet passes, comprising at least a water flow agitation system, also referred to in the present specification as a flow accelerator, located at the exit of one of the curves towards the contiguous straight segment of the channel.

Following the teachings in the prior art in order to achieve hydraulic improvements, at least one of the two curves, and specifically, the curve contiguous to the flow accelerator, is 2 to 4 times narrower than the straight segment of the channel (B), and has an average curvature radius (R) 1 to 2 times greater than the width of the curve itself, and is joined to the end of the straight segment located at the entry thereof by means of a transition region, which is a narrowing with flat walls with the shape of an isosceles trapezoid, wherein the longest of the two parallel sides corresponds to the width of the straight segment and the shortest to the width of the entry of the curve, presenting a depth increase in the same proportion as the narrowing of its width with respect to the straight segment, and a length such that the ratio between said length and the width of the straight segment of the channel is comprised between 1 and 3.

This way, the cross sectional area of the transition region (also referred to in this specification as the narrowing region) and the area of the curve itself are constant and equal at all times to the area of the straight segments of the channel, such that the flow speed is also kept constant and at typical values comprised between 0.2 and 0.4 m/s. That is to say, the configuration of the flow channel, both in terms of the curve and the introduction of a transition region between the curve and the straight segment means that there will not be significant variations in the different sections thereof, and in consequence, in the flow speeds, such that the hydrodynamics of the system remain unaltered. In addition, by presenting straight planes in the walls and in the bottom, the construction process becomes easier. This design allows minimizing the load loss and the dead regions. In addition, the depth of the proposed curve allows a simple transition to the agitation system. Likewise, at the outlet of the agitation system towards the straight segment, a second transition region, as the one described above, is included between the curve and the inlet into the agitation system.

According to the prior art, this HRAP reactor design with an improved curve and transitions between segments presents the following configuration: a first, conventional, curve; a first straight segment of the channel; a first transition region; a second, improved, curve; a flow acceleration system; a second transition region; and a second straight segment of the channel up to the first, conventional, curve. This configuration reduces the agitation energy by using at least one improved curve, in addition to the new flow acceleration system. This way, the invention presents the essential characteristic of taking advantage of the depth achieved at the transition regions, and that are required by the proposed flow acceleration system to insert the curve, given that it has similar geometric characteristics in terms of depth and width to the flow acceleration system. It could be said, therefore, that the main improvement of this invention (new agitation system) is coupled in geometric and hydraulic terms with the configuration of the curve of the channel, and therefore, the same transition necessary for one of the elements can also be used for the other, with the resulting reduction in construction costs.

In short, in order to optimize the productivity of the algal biomass, the configuration of the aforementioned curve minimized the dead regions with respect to a conventional curve, and therefore, the loss of algal biomass in the associated solid settling process.

According to the present invention, in order to achieve the advantages intended with respect to the improvement in the agitation of the water flow, the agitation system that comprises the reactor is located between two transition regions, one at the inlet thereof and contiguous to the curve, and the other at the outlet thereof and contiguous to one of the straight segments of the channel, consisting of a propeller flow acceleration device (commercial, widely used in wastewater treatment plants), with an axial flow production at a rotational speed equal to or lower than 100 rpm. The design of the blades of these propellers allows directing an elevated flow rate in the axial direction based on a low consumption of energy, thereby obtaining a global agitation performance between 70% and 80%. The propeller is inserted into a closed conduit with tubular walls, with two open ends that allow the passage of the entire water sheet through its interior, located below the level of the water sheet (that is to say, the highest portion or point thereof is located at a height below the water level in the channel, which is able to prevent the vortex effect and also minimize the backflow from the propulsion at the exit of one of the ends of the conduit to the aspiration at the opposite end of the conduit, thanks to an inner diameter of the tubular conduit that allows small clearances with respect to the propeller, between 2 and 20 mm between the end of the propellers and the inner surface of the wall of the conduit (in other words: the difference between the inner diameter of the tubular conduit and the diameter of the propellers is comprised between 4 and 40 mm, that is to say, twice the space). This point is crucial, given that it was possible to prove that, if said clearance were larger, there would be a considerable hydraulic loss caused by backflow at the ring section.

The design of the tubular conduit contained by the flow agitation device is defined by a minimum load loss, thanks to transitions without dead regions or detachment of flow at the walls from the exit of the curve and by maintaining the cross sectional area approximately constant and with a value close to the rest of the HRAP. With these design criteria in mind, the average flow speeds in all of the elements of the HRAP (agitation system, transitions, curves and straight channel) are kept approximately constant and with values between 0.2 and 0.4 m/s, according to the design value selected.

The open HRAP reactor, object of the present invention, achieved a reduction in the consumption of agitation energy and an optimization of the productivity of algal biomass. Specifically, it was able to reduce the consumption of agitation energy between 80% and 90% with respect to conventional paddlewheel systems. Likewise, as it was affirmed above, the configuration of the curve minimizes dead regions with uncontrolled settling and therefore, the associated loss of algal biomass. The more efficient agitation system generates a homogenous flow in the entire channel, also preventing uncontrolled settling at the bottom of the HRAP, which is usual in conventional systems.

In short, there are multiple advantages derived from the present invention. Specifically, the known advantages brought by the curve design according to the prior art to the energetic efficiency and the productivity of the reactor are the following:

- The curved design presented load loss coefficients between 0.3 and 0.45, lower than those provided by curves with conventional deflectors K~2 (Lundquist, T.J., 2010). Therefore, a reduction of energy above 75% was obtained. This reduction is due to improved hydraulics, with a ratio between the average curvature radius R and the width of the channel B between 1 and 2. On the other hand, the greater depth of the curve design, in comparison with conventional curves, allowed a simple adaptation to the new agitation system or flow acceleration system by having to overcome a lower incline with respect to the bottom of the flow acceleration system, thereby simplifying the transitions towards the flow accelerator.
- Finally, the narrowing and widening before the curve had lower load losses than the straight segment of the channel equivalent to the same length, which supposes additional energy savings estimated at 6.3% for significant transition lengths, such as the length of Example 2.

Apart from that, some improvements are shown with respect to the Liffman, Paterson et al. (2013) reference, which may serve as reference, are the following:

- The transition region between the straight segment of the channel and the entry of the curve is made at lower construction costs, given that the walls and bottom in the present invention are inclined straight planes and not curved, due to which they are not defined by the cosine function proposed in the article by Liffman, Paterson et al.
- Said transition (or narrowing) region has the shape of an isosceles trapezoid and is symmetrical with respect to the central axis of the main straight channel of the HRAP. This symmetry allows centering the new agitation or flow acceleration system with respect to the axis of the straight segment of the channel and not in its external portion, allowing a uniform distribution of flow throughout its entire wideness.

The agitation system according to the present invention uses submersible agitators with low rotational speeds (<100 rpm). These agitators or flow accelerators are constituted by gear motors and axial propellers with two or three blades and are energetically optimized to drive a large water flow in an axial manner. These flow accelerators have been used in wastewater treatment facilities, mainly in carrousel or oxidation channel of wastewater treatment facilities. In these oval channels, with the shape of a circular rectangle, water is circulated in the open flow channel at a horizontal speed between 0.2 and 0.4 m/s, similar to the speed of a conventional HRAP reactor for the cultivation of microalgae. However, there is an important difference, consisting of the fact that the carrousels or oxidation channels of the wastewater treatment facilities usually have water sheet depths above 3 meters, and the present case is designed to have depths between 0.2 and 0.4 meters (which is the depth used in the main straight channels of conventional HRAP reactors, because it is the appropriate depth for the growth of algae in this type of reactors), insufficient for the operation of the equipment. Adapting these agitators to work at this shallow depth between 0.2 and 0.4 m has been the chief technological challenge of the present invention and its main novelty, given that this equipment has never been used under these working conditions and even less so in this type of reactors.

Besides, according to the present invention as defined in claim 1, the optimized design of the agitation system consists of inserting the agitation device into a closed wall tubular conduit, open at its ends, to allow the axial passage of the entire water sheet through its interior, located between two transition sections (which may be built in different materials, such as metalwork or PRFV polyester), the first one joining the circular section of the conduit with the rectangular section of the curve on one end, and the second transition section joining the circular section of the conduit with the section of a second transition region of the channel at the other end, with the same configuration as the first, that is also joined to the contiguous straight segment of the channel. As explained above, the flow acceleration system is sized with a cross sectional area of the same order of magnitude as the remaining course of the HRAP reactor (straight channel, transition and curves). Therefore, the agitator works as a "flow accelerator" with flow speeds comprised between 0.2 and 0.4 m/s, speeds that are maintained throughout the entire HRAP. Flow accelerators have, in turn, the following advantages with respect to conventional paddlewheel agitation systems:

a) They are optimized commercial devices existing in the market with a broadly extended commercial and after-sale network at an international level with numerous manufacturers, in comparison with paddlewheel devices, which are handcrafted fabrications that are custom-made for every occasion without a commercial and after-sale network thereof, which makes the initial investment and maintenance costs, as well as the quality of the equipment, more expensive.

b) Better hydraulic and global energetic performance. Manufacturers contribute energetic hydraulic performance values above 95%, and between 75% and 80% of the global energetic performance of the system. There is an important dispersion of data in the paddlewheel. If we estimate an approximate average of the reported values of 20%, a reduction in consumption of approximately 75% was obtained with the flow accelerator, only for this concept, without taking into account the other hydraulic and energetic improvements of the invention.

With respect to high-speed submersible agitators, which have been used in some occasions as an improvement alternative to the paddlewheel, it should be noted that the low-speed agitators proposed in the present invention have a much larger diameter than the high-speed agitators (between 900-4,000 mm vs. 100-400 mm) for the same flow rate, given that they work at fewer rotations (<100 rpm vs. >700 rpm). The advantages of using low-speed agitators (<100 rpm) with large diameters are the following:

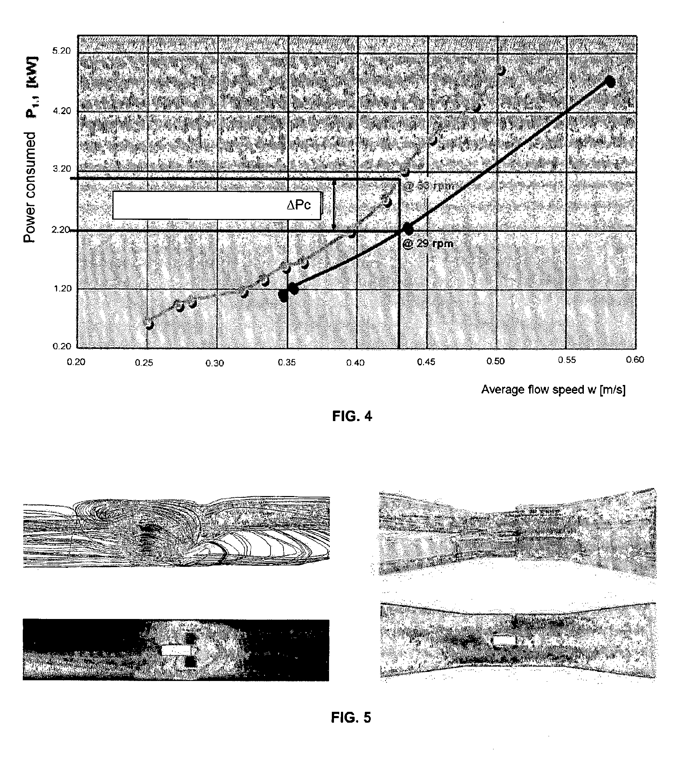

a) Higher hydraulic efficiency. Flow accelerators with large diameters and low revolutions (<100 rpm) are equipments that are far more efficient energetically than high-speed agitators with smaller diameters (> 700 rpm) for the same flow rate supplied. This energetic efficiency is verified with a power factor of these low-speed agitators presenting a value between 600 and 1,200 N/kw, which are values 4 times higher than the 100-300 N/kw of high-speed agitators. This fact can be understood with the graph of Figure 4 (state of the art), corresponding to a manufacturer of commercial accelerators (WILO-EMU), wherein two agitators with different diameters and rotational speeds are compared. The Figure shows that the power consumption of the agitator with the larger diameter (2,500 mm vs. 2,100 mm) and fewer revolutions (29 rpm vs. 53 rpm) is 30% lower for the same flow speed, which indicates the higher energetic efficiency of the equipment with the larger diameter.

b) Lower load losses. In the present invention, the closed conduit where the flow accelerator is located was designed with a constant cross sectional area and a value close to the value of the rest of the HRAP (straight channel, transitions and curves), such that the speed is kept low and with the same values between 0.2 and 0.4 m/s. The foregoing is possible because the low-speed agitators are able to work at these flow speeds, below 1 m/s. In addition, by designing the conduit in this manner, the contractions, expansions and turbulences of the flow of high-speed submersible agitators were prevented, as well as their associated load losses.

c) Lower cell stress: by working with fewer revolutions (<100 rpm), algae cells are treated in a gentle manner, unlike the high-speed agitators that produce stress, especially in some flagellated or filamentous species.

d) The vortex effect and cavitation are prevented, given that the accelerator device is inserted into a closed tubular conduit (with a circular section) and not in an open rectangular channel. This last option requires a high submergence (water sheet over the agitator) to avoid this problem.

e) Increase of the volumetric performance (measured as the ratio between the real effective flow rate in HRAP and the flow rate propelled by the propeller) at values above 95%, with respect to 50% values, produced in an open rectangular channel. In other words, in the design proposed in the invention, the real effective flow rate in HRAP presents values that are close to the values propelled by the propeller. This is due to the fact that the proposed design leads the flow within a tubular or circular conduit, with small clearances between the propeller and the tube (approximately comprised between 2 and 20 mm). In open rectangular channels, however, there are parasite flows or backflows between the higher pressure of the propulsion and the lower pressure of the aspiration, through a large clearance gap existing between the propeller and the channel, thereby reducing the real effective flow rate in HRAP.

The most important effect to be considered in terms of the configuration of the reactor presented herein, constituted by the designed agitation system, is the synergy between said system and the curve in the same reactor, and especially by having the contiguous arrangement defined herein. Thus, the main advantages presented by both elements of the reactor are the lower consumption of hydraulic energy (see Tables 3 and 8 below), such as the load loss of the curves, and the reduction in the consumption of agitation energy. However, it should not be forgotten that the configuration of the curve and the flow acceleration system, especially in terms of its depth that allows maintaining constant the area of all the sections, produces synergic effects that foster this reduction in the consumption of energy and a greater production of biomass: the agitator needs a certain depth due to its diameter (in Example 1 presented herein, this depth reaches approximately 900 mm, being the double 1,800 mm, in Example 2), this depth being a priori significantly higher than the depth of the main straight channel segments, which really acts as the reactor and usually presents a depth of 300 mm, given that algae need little depth in order to allow the passage of light in the water sheet where they are cultivated. The development of the optimal agitation system, as described herein, required the reconfiguration of the other cross sections, such as the curves, and it was observed that the existence of two transition sections at the inlet and at the outlet of said agitation system, allows changing the depth between sections without altering the flow speed because the area is kept constant, which is also strengthened by the configuration of the contiguous curve. According to the invention as defined in claim 1, the first transition section described is located at the inlet of the agitation system, before the tubular conduit, and the second section is located at the outlet of the tubular conduit, where the flow accelerator propels the water flow.

Before the definition of the final configuration of the open reactor, object of the present invention, fluid-dynamic simulations with FCD techniques were carried out, in which it was possible to verify, on a comparison basis, the speed lines of an agitation device in an open conduit, in addition to the perfectly guided flow of the tubular conduit of the invention. This way, the real or net effective flow rate in HRAP goes from a 50% of the flow rate propelled by the propeller in the open channel to a value above 95% in the invention (Figure 5).

## Detailed description of the invention

Preferred embodiments of the high rate algae pond reactor are defined in the subclaims 2 to 13 as well as in the description. In a preferred manner, the width of the two straight segments (B1) is comprised between 0.5 and 25 m, and the depth of the water sheet is comprised between 0.2 and 0.6 m. This is important to the extent its section is kept constant throughout the entire course, as required for the present invention.

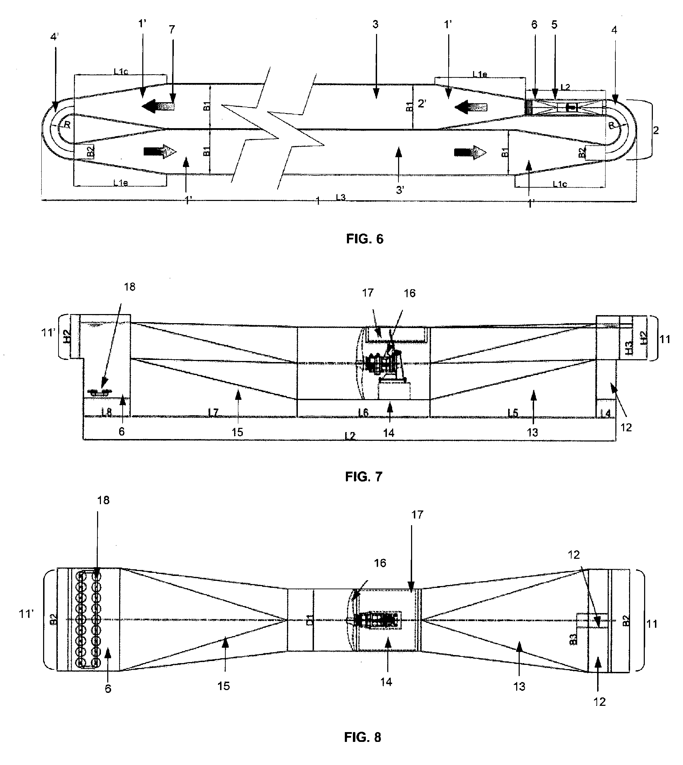

In a preferred case of the invention, the reactor only contains one agitation system, but the two curves of the open HRAP reactor present the configuration previously defined, and both are joined at their two ends to the straight segments by means of two transition regions, such that the reactor comprises four transition regions, two for each curve. This is the embodiment shown in Figure 6 (plant view) and defined in claim 3. In this case, the configuration of the channel is the following: first improved curve; first transition region; first straight segment; second transition region; second improved curve; flow acceleration system; third transition region; second straight segment of the channel; fourth transition region up to the first improved curve. In this case, we have the advantages arising from the new configuration of the curve contiguous to the agitation system, plus the hydraulic improvements arising from the replacement of the other conventional curve with the improved curve following the teachings in the prior art.

Also in a preferred manner, at least one of the curves, although it might be both of them, comprise a deflector with the same curvature radius than the curve itself, which allows improving the laminar conditions of the flow. These conditions are important with respect to the minimization of the load losses and the correct operation of the flow acceleration system.

In an even more preferred case, as defined in claim 4, the HRAP reactor does not only present two curves with a configuration improved according to the teachings in the prior art, but also two flow acceleration systems as the one described above, one at the outlet of each one of the two curves of the channel. Therefore, in the case in which the length of the reactor requires it so (determined by the length of its straight segments), two flow accelerations can be installed, one next to each curve, which also present the new configuration defined in the present invention.

The agitation system (illustrated in Figures 6 and 7) contains a circular tube wherein the flow accelerator is concentrically housed. Preferably, the length of the tube or conduit with a circular wall is sufficient to normalize the horizontal (axial) flow before and after the flow accelerator and to facilitate the maintenance thereof. Even more preferably, said length is comprised between 1 m and 5 m, sufficient to normalize the horizontal (axial) flow. This length allows the placement of manholes enabling the access to the conduit. The circular tube presents a diameter D1 that is adjusted to the diameter of the agitator housed inside, preferably with 10 mm clearance, which should be understood as the distance between the end of the blade/propeller of the acceleration device and the inner wall of the circular conduit.

The circular conduit must be completely filled with water, without air pockets, due to which the upper portion of said conduit (which must be understood as the highest point of the outer wall of the tubular conduit) is always located at a height (H4) below the lower level of the water sheet in the channel (lower level it must be understood as the greater depth of the sheet in the channel, which at this point corresponds to the depth of 0 m at the inlet of the agitation system). Said height can be preferably comprised, but not limited to, 100 and 400 mm below the level of the water sheet.

The flow acceleration device is a conventional agitator as the one offered by several manufacturers, with a variable propeller/blade diameter, for example, in the present case but without being limitative, 900 mm, such as in Example 1, or more preferably 1.8 m, as illustrated in Example 2. Also preferably, the rotational speed is comprised between 30 and 80 rpm. A CFD analysis, as those carried out for the design of the invention, allowed to calculate the load losses in the entire HRAP reactor in order to select the power required for the agitator.

As an option, but nevertheless significantly improving the performance of the entire reactor, the agitation system can further comprise at the inlet thereof a (solid) settling pit, which avoids the accumulation of solids throughout the entire reactor, but is also very convenient in cultures with the presence of sands and other settleable solids at the ordinary speeds of the reactor (between 0.2 and 0.4 m/s). It is convenient to locate the settling pit just before the acceleration conduit to avoid the entry of solids into the same and the accumulation of said solids at the lower height where the propeller is located, which could cause operating problems. This way, uncontrolled settling is prevented at the bottom of the HRAP channel, which is a usual problem in conventional systems, thanks to the design of the settling pit for settleable solids that can be located at the inlet of the agitation system or flow accelerator. This solids settling pit, which can be located at the exit of the curve and at the inlet of the agitation or flow acceleration system, avoids the accumulation of the same in the HRAP by means of a controlled settling of solids and their extraction, thereby maintaining the stability of the culture over time.

The settling pit of the agitation system is located between the contiguous curve to which said system is joined (that is to say, at the entry thereof) and the inlet of the tubular conduit located inside the agitation system itself. Preferably, this controlled solid settling region (referred to as L4 in the Figures) has a rectangular shape in the upper portion with a length L4 (for example, between 0.5 and 1 m) and a width B2 (equal to the curve), and a horizontal flat bottom with a rectangular shape with a length L4 (equal to the upper portion) and with a width B3 (for example between 0.4 and 0.6 m) in the lower portion that matches the lower portion of the tubular conduit, both sections being joined by inclined planes to facilitate the descending displacement of the solids. At the flat, horizontal bottom, a submersible pump can be preferably placed to extract settled solids. The horizontal flat bottom matches the minimum level of the tubular conduit (referred to in the Figures as Z6) to facilitate the construction and support of the entire system during the installation thereof during the works.

Next to the solid settling pit described above, it is located the first transition section of the agitation system as described above, specifically between said settling region and the inlet of the circular conduit in which the flow accelerator is located. In this preferred embodiment, the first transition section consists of a closed conduit (preferably constituted by a hopper) for the transition between a rectangle with a height H2 and a width B2 corresponding to the section of the curve, and a circle with a diameter D1 corresponding to the section of the tubular conduit, formed by four triangles and four curved segments. This first transition section presents downslopes comprised between 1:4 and 1:6, and once again, maintains the cross section of the flow channel constant. To achieve the foregoing, the diameter D1 of the circle is initially calculated such that its circular area is equal to the cross section in the curves and is adjusted depending on the commercial flow accelerators available, based on this value, and taking into account the clearance that the inner surface of the tubular conduit must present relative to the end of the propellers/blades of the acceleration device.

Besides, in this preferred embodiment, the second transition section, located between the outlet of the (central) tubular conduit and the contiguous transition region, located at the outlet of the agitation system towards the contiguous straight segment, is equal to the first one.

Also preferably, with or without a settling pit, the agitation system can further comprise a carbonation system. In Figures 7 and 8, said agitation system, having a length L2, includes the carbonation system. This carbonation system, referred to as L8 in figures 7 and 8, is housed within the agitation system, specifically between the outlet of the tubular conduit, housing the agitation device and the transition region located at the outlet of the agitation system, connecting the same to the contiguous straight segment. Said carbonation system is, in the most preferred embodiment, a system as the one described in the Spanish patent application P201231485 (HIDROTEC TECNOLOGÍA DEL AGUA, S.A.), which eliminates the conventional vertical deflectors, and therefore, the high consumption of energy associated with the vertical deflector, which is the main load loss in conventional HRAP reactors (K~4). The configuration of this carbonation system is fundamentally characterized in that it has a pit, having a width equivalent to the width of the flow channel and a length comprised between half and the double of the width of the channel, and in that it comprises at least one gas injection device inside the pit, located at the base thereof and next to the wall opposite to the incoming direction of the water flow, with a distance from the upper face of the device through which the gas is injected to the bottom of the flow channel comprised between 0.5 and 1.5 m, including both limits. It should be noted that even though the carbonation system is designed according to the criteria reflected in the Spanish patent application P201231485, given that it is a narrower channel than the straight segment of the HRAP channel, (in Example 2 it is 3 m instead of 9 m), the area of the horizontal section of the carbonation pit must be maintained, by extending the length thereof proportionately, therefore increasing from 60 cm to 180 cm.

It should be noted that the section of the channel is kept constant throughout the length of all its sections for the water not to suffer any accelerations or decelerations that increase the consumption of energy, except for the solid settling pit and the carbonation system contained in the agitation system. These elements have a greater cross-sectional area than the rest to enable both, the settling and the carbonation process.

In the most preferred case of all, the HRAP reactor is composed of the following elements, according to the example shown in Figures 6, 7 and 8:

a) two straight segments of the channel (3, 3') with a width B1 (2'), comprised between 0.5 and 25 m, and with a water sheet depth H1 between 0.2 and 0.6 m. The cross section of the straight segment, which is kept constant in the other sections to have a constant flow, is defined as $S = B1 \times H1$. In the channel of Example 2, B1 = 9 m and H1 = 0.3 m, the cross sectional area would be $S = 9 \text{ m} \times 0.3 \text{ m} = 2.7 \text{ m}^2$;

b) two curves (4, 4') at the ends of the straight segments of the channel (3, 3'), with an average curvature radius R and width of the channel B2. The R/B2 ratio is between 1 and 2. The cross section of the curves (4, 4') is also kept constant. In Example 2, the cross section would be equivalent to $S = B2 \times H2 = 3 \text{ m} \times 0.9 \text{ m} = 2.7 \text{ m}^2$, equal, therefore, to the section of the two straight segments (3, 3'). In a preferred case, the curves (4, 4') with R/B2 < 1.5 will have a deflector with a radius R;

c) four transition regions or narrowings (1'), with an isosceles trapezoid shape, between the straight segment of the channel (3, 3') with a width B1 and the curve (4, 4') with a width B2, in lengths L1e (narrowing region or outlet of the water flow of the curve) and L1c (contraction region or inlet of the water flow of the curve). This narrowing (1') maintains a constant cross section S, as the cross section of the straight segment (3, 3'), thereby decreasing the width in the same proportion that the depth H increases along the length L of the narrowings (1'). The B1/B2 ratio presents values between 2 and 4. Therefore, the depth H increases at the same value along the length L1. In Example 2, for a straight segment of the channel (3, 3') with a width B1 = 9 m and for a curve (4, 4') with a width B2 = 3 m (B1/B2 = 3), the depth of the water sheet will go from H1 = 0.3 m in the main channel (3, 3') to H2 = 0.3 m x 3 m = 0.9 m at the curve (4, 4').

The length L1 of the transition region (1') is calculated with respect to the width of the straight segment of the channel (3, 3') with a width B1, such that the L1/B1 ratio ranges between 1 and 3. These transitions (1') have a lower load loss than the equivalent straight channel (3, 3'), due to which, in Example 2, a ratio close to the upper value has been chosen, where L1= 24 m and L1/B1 = 2.66.

d) an agitation system (5) with a flow accelerator (16) located inside a tubular conduit (14) with two open ends for the passage of the water flow, located in the central portion of the agitation system (5) with a length L2, which includes a sedimentation or disposal pit (12) for settled solids, and preferably a carbonation system (6) as well. The agitation system (5) or flow accelerator of Figure 8 shows the different regions it presents, in a more detailed manner, with the purpose of illustrating the invention in one of its embodiments. However, it should not be considered a limitative embodiment thereof.

- A controlled solid settling region (12) or pit, with a length L4 (between 0.5 and 1 m), and with a width B2 in the upper portion and horizontal flat bottom with a width B3 (from 40 to 60 cm) in the lower portion, both of them joined by inclined planes to facilitate the downwards displacement of the solids. A submersible pump can be placed in the horizontal flat bottom for the extraction of the settled solids. The horizontal flat bottom matches the minimum level of the tubular conduit to facilitate the construction and support of the entire system during the installation thereof, during the works.

- A transition section (13) between the settling region (12) and the circular conduit (14) where the flow accelerator (15) is located. It is a closed conduit constituted by the typical hopper made with metalwork techniques for the transition between a rectangle (H2xB2) and a circle (diameter D1), formed by 4 triangles and four curved segments. Once again, this transition section maintains its cross section substantially constant. To achieve the foregoing, the diameter D1 is initially calculated such that its circular area is the same than the cross sectional area in the curves. In Example 2:

$$S = H2 \times B2 = 3\ m \times 0.9\ m = 2.7\ m^2$$

The diameter D1 obtained as provided above would be: $\sqrt{S \times \frac{4}{\pi}} = 1.85\ m.$ Anyway, this diameter must be adjusted depending on the commercially available flow accelerators that are close to this value. For Example 2, a commercial agitator with a diameter = 1.8 m was selected, and the D1 value (circular section of the conduit) is = 1.82 m, with a clearance of 10 mm between the end of the propeller and the conduit. The length L5 is calculated with downslopes of approximately 1:4 to 1:6.

- a tubular conduit (14), which is a circular tube with a diameter D1 wherein the flow accelerator (15) is concentrically located, preferably with a clearance comprised between 2 and 20 mm between the end of the blade/propeller of the agitator device and the inner surface of the circular conduit. The length L6 has a value between 1 m and 5 m, sufficient to normalize the horizontal flow before and after the flow accelerator (15) and for maintenance purposes. To achieve the foregoing, manholes (17) can be placed to allow the access to the conduit (14). The circular conduit is completely filled with water, with no air pockets.
- a second transition section (15) between the outlet of the tubular conduit (14) and the carbonation system (6). It consists of a hopper similar to the first transition section (13), with a length L5; and
- a carbonation system (6). This system is designed according to the criteria reflected in patent application P201231485. However, even though it is a channel that is narrower than the straight segment of the main channel of the HRAP (in Example 2, it is 3 m instead of 9 m), the area of the horizontal section of the carbonation ditch (6) is maintained by extending the length thereof proportionately, increasing therefore from 60 cm to 180 cm.

**Brief description of the Figures**

Figures 1 (state of the art): a conventional design of a HRAP installation for the cultivation of algae including the main energy-consuming elements (state of the art):

  1. Total length of the HRAP installation
  2. Total width of the HRAP installation
  3. First main straight segment
  3'. Second main straight segment
  4. First curve (with deflectors)
  4'. Second curve (with deflectors)
  5. Agitation system (paddlewheel)
  6. Carbonation systems (one at each straight segment).

Figure 2 (state of the art): HRAP installation design proposed by Liffman (2013) with drop-shaped curves:

  1'. Length of the exit of the curve
  2'. Width of the straight channel segment of the HRAP installation 5. Agitation system (paddlewheel)
  7. Culture water flow
  8. Narrowing of the flow channel at the exit of the curve defined by the cos(X) function.

Figure 3 (state of the art): cross-sectional view of a paddlewheel agitation system.

  5. Paddlewheel agitation system

9. Direction of the rotation of the paddlewheel agitation system
10. Water sheet

Figure 4. Relation between the flow speed in m/s (axis of abscissas) and the power consumed in kw (axis of ordinates) of two compared commercial agitators (WILO-EMU). The ∆Pc variable represents the increase (or difference) of the power consumed among agitators. The light colored line represents a commercial agitator model TR221, with a diameter of 2,100 mm, while the dark colored line represents a commercial agitator model TR225, with a diameter of 2,500 mm.

Figure 5. Traces and speed profiles (represented by the gradation of grays, where speed decreases as the color gets darker) of a comparative fluid-dynamic simulation between an agitation device located in an open rectangular channel (image to the left) and an agitation device located in a closed tubular conduit of the present invention (image to the right).

Figure 6. Plant view of a preferred embodiment of the open reactor for the cultivation of algae object of the present invention, wherein the two curves present the same configuration defined above and in the two ends of each curve a transition region towards the straight region is included. In one of the curves, as defined in the invention, the new agitation system between the exit of the curve and the transition region is included.

1. HRAP or raceway installation with a total length L3
1'. Transition region between the exit of the curve 4 and 4' and the contiguous straight segment of the channel 3 and 3', with a length L1c in the narrowing transition regions of the flow channel, and L1e in the widening transition regions of the flow channel.
2. Total width of the curve.
3. First main straight segment, with a width 2' (B1)
3'. Second main straight segment, with a width 2' (B1) 4. First curve, with a width B2 and a curvature radius R
4'. Second curve, with a width B2 and a curvature radius R
5. Agitation system with flow accelerator, with a length L2
6. Carbonation systems included at the outlet of the agitation system 5
7. Culture water flow

Figure 7. Elevational view of the agitation or flow acceleration system (5), comprised in the reactor of one of the preferred case of the present invention, which presents a solid settling region and a carbonation system.

6. Carbonation system
11. Entry of the water flow from the curve contiguous to the agitation system, with a height H2.
12. Controlled solids settling region or pit, with a length L4.
13. First transition section, between the settling region 12 and the tubular conduit 14 wherein the flow accelerator 15 is housed, with a length L5.
14. Tubular conduit, with a length L6, wherein the flow accelerator 16 is housed.
15. Second transition section, between the outlet of the tubular conduit 14, wherein the flow accelerator 16 is housed, and the carbonation system 6, with a length L7.
16. Agitator or flow accelerator
17. Manhole to access the tubular conduit 14
18. Carbonation gas injector(s)

Figure 8. Plant view of the agitation or flow acceleration system comprised in the reactor of the present invention and shown in Figure 7, presenting a solids settling region and carbonation system.

6. Carbonation system
11. Inlet of the water flow from the curve 4 contiguous to the agitation system 5, with a width B2.
11'. Outlet of the water flow from the agitation system 5 to the transition region 1', with a width B2.
12. Controlled solids settling region or pit, with a horizontal flat bottom with a width B3 (12').
13. First transition section, between the settling region 12 and the tubular conduit 14, wherein the flow accelerator 15 is housed.
14. Tubular conduit, with a width D1, wherein the flow accelerator 15 is housed.
15. Agitator or flow accelerator
16. Second transition section, between the outlet of the tubular conduit 14, wherein the flow accelerator 15 is housed, and the carbonation system 6.
17. Manhole to access the tubular conduit 14.

18. Carbonation gas injector(s)

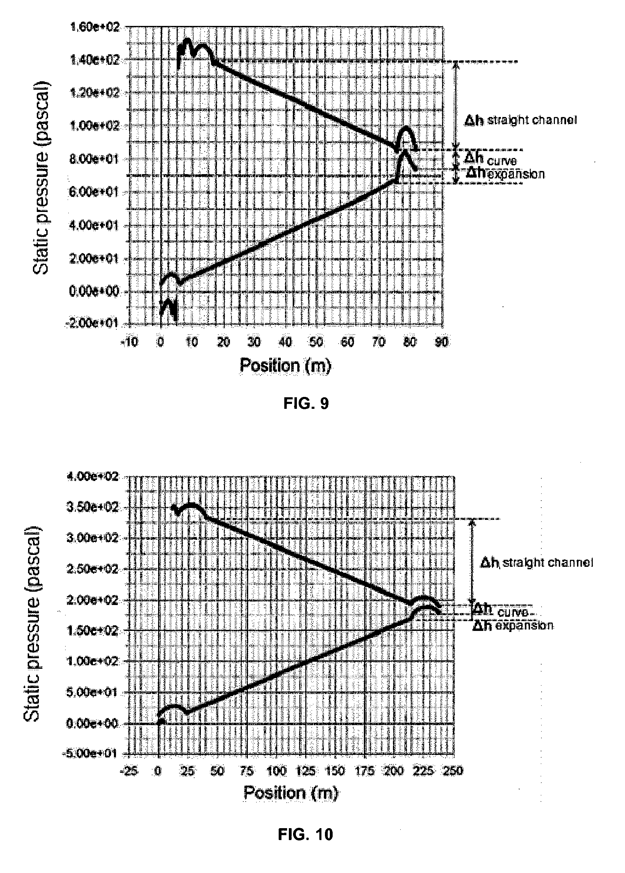

Figure 9. Load losses of the HRAP reactor object of the invention, corresponding to Example 1. The straight light and dark colored lines represent the static pressure along the two main straight segments, $\Delta h_{straight}$ channel being the load loss throughout the straight segment and calculated as the difference between the end points. $\Delta h_{expansion}$ represents the load loss at one of the transitions joined to a straight segment.

Figure 10. Load losses of the HRAP reactor object of the invention, corresponding to Example 2. The straight light and dark colored lines represent the static pressure along the two main straight segments of the flow channel, $\Delta h_{straight}$ channel being the load loss throughout the straight segment and calculated as the difference between the end points. $\Delta h_{expansion}$ represents the load loss at one of the transitions joined to a straight segment.

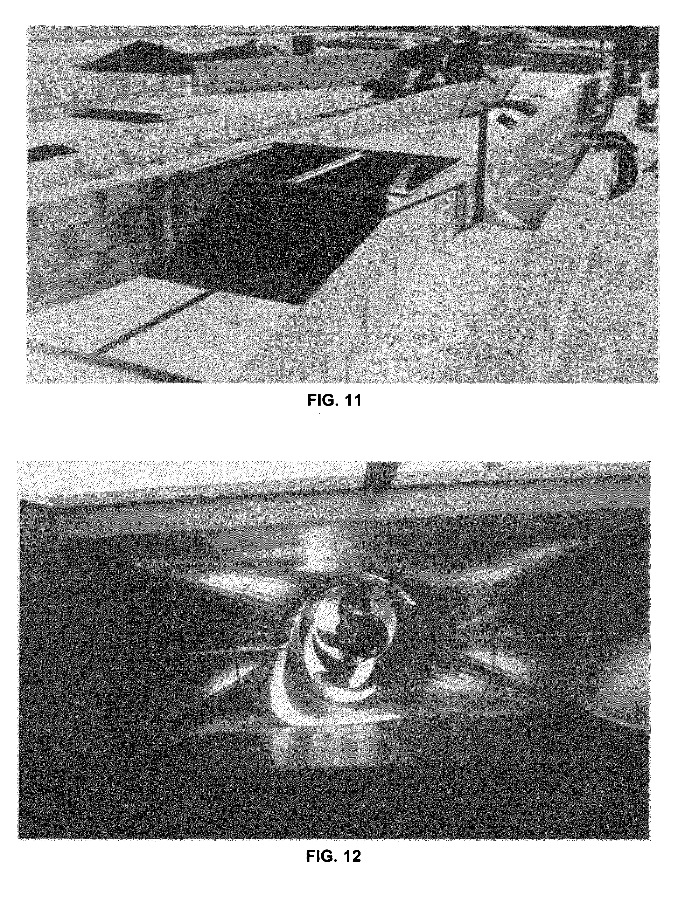

Figure 11. View of the agitation system from the exterior of the flow channel, constituted by the tubular conduit located between two hoppers made with metalwork techniques constituting the two transition sections.

Figure 12. Frontal view of the interior of the agitation system, showing the flow acceleration device inside the tubular conduit from the second transition section.

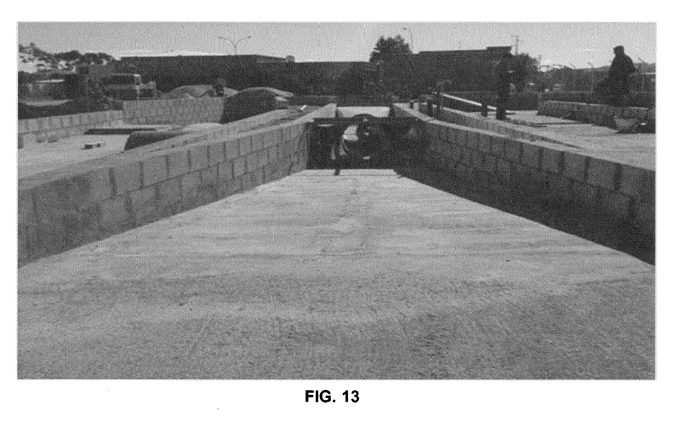

Figure 13. Frontal view of the agitation system from the transition region between the outlet thereof and the first main straight segment. The isosceles trapezoid shape of the transition region and the way in which the depth decreases to the extent the width of the channel increases from the outlet of the agitation system to the contiguous straight segment, can be appreciated.

**Examples**

Example 1. Design and configuration of a reactor according to the present invention. Measurement of performance and productivity

A fluid-dynamic simulation by means of CFD techniques in 3D was carried out with the *ANSY fluent* program of a pilot 500 $m^2$-HRAP according to the design of the invention until the optimization of the parameters. Subsequently, a full-scale pilot was made for the validation of the results.

The resulting parameters after the optimization, according to the configuration of the reactor shown in Figures 6, 7 and 8, are the following:

H1 = 0.3 m (height/depth of the sheet in the straight segment of the channel B1)
B1 = 3 m (width of the straight segment of the channel)
B2 = 1.5 (width of the curve)
H2 = 0.6 m
R = 1.5 m (curvature radius of the curve)
L2 = 11 m (length of the agitation system)
L1 = 6 m (length of the transition region between the curve and straight segment)
L3 = 85.5 m (total length of the reactor)
L4 = 0.5 (length of the settling region within the agitation system)
L5 = 3 m (length of the transition section between the settling region and the tubular conduit housing the flow accelerator)
L6 = 3.5 m (length of the tubular conduit)
L7 = 3 m (length of the second transition section between the tubular conduit and the carbonation system)
L8 = 1 m (length of the carbonation system)
D1 = 920 mm (diameter of the tubular conduit)
D = 900 mm (total diameter of the flow accelerator)
H3 = 100 mm

The agitator selected is an ABS SB900 model, with a diameter of 900 mm and low output speed (79 rpm), but with a high global performance of 78%, according to the data provided by the manufacturer.

The other design parameters are those contained in Table 1.

**Table 1. Design parameters of the 500 $m^2$-HRAP reactor**

| STARTING DATA | Design of the invention | Conventional |
|---|---|---|
| Speed (m/s) | 0.25 | 0.25 |
| Density (kg/$m^3$) | 998.4 | 998.4 |

(continued)

| STARTING DATA | Design of the invention | Conventional |
|---|---|---|
| $L_{contractions and expan}$ L1 (m) | 6 | |
| Roughness (mm) | 0.003 | 0.003 |
| Area (m$^2$) | 500 | 500 |

Based on the previous design parameters, the fluid-dynamic simulation allows obtaining the load losses h along the system as presented in Figure 9. Based on the load losses h calculated with the mathematical simulation, the localized loss coefficient K can be obtained according to the following formula:

$$h = K \times \frac{v^2}{2g}$$

These values are presented in Table 2, which was completed with the values of the bibliographic loss coefficients referenced above for the conventional system. For the purposes of comparison, the load loss values of the equivalent straight channel with a length L1 = 6 m were calculated, which resulted in a value equivalent to K contraction region = K expansion region = 0.160. This value is 25% higher than the average K value of the expansions and contractions of the new design due to the larger hydraulic radius. The foregoing supposes an additional hydraulic improvement in the proposed system.

**Table 2. Load losses Coefficients**

| Loss coefficients | Deign of the invention | Conventional |
|---|---|---|
| $K_{curves}$ | 0.44 | 2 |
| $K_{expansion regions}$ (L = 6m) | 0.18 | 0.160 |
| $K_{contraction region}$ (L = 6m) | 0.08 | 0.160 |
| $K_{carbonation pit}$ | | 4 |

Based on the previous K values, it is possible to estimate the hydraulic improvements represented by the new configuration of the reactor, estimated as the reduction of the load loss in the entire reactor. The load losses for the conventional system were calculated by applying the Darcy equation after calculating the friction factor by means of the Colebrook-White equation, resolved using the Newton method up to a tolerance of 1e-16. It was possible to verify that this system provides the same values than the fluid-dynamic simulation. The results are presented in Table 3.

**Table 3. Load losses and Hydraulic Improvements of the New Design**

| 500 m$^2$ Pond | $h_{straight+accessories}$ (m) | $h_{curves}$ (m) | $h_{total}$ (m) |
|---|---|---|---|
| Conventional design | 0.026650 | 0.012750 | 0.039400 |
| Proposed design | 0.013500 | 0.002810 | 0.016310 |
| HYDRAULIC IMPROVEMENTS | | | 59% |

According to Table 3, and given that the consumption of energy P (w) is proportional to the load loss h (m) according to the formula:

$$P = \frac{\rho \times Q \times g \times h}{\eta}$$

it was deducted that the new design represented a reduction in the consumption of energy due to hydraulic improvements of 59%.
However, the present invention also represents a reduction of energy due to the agitation system. The consumption of energy P in the entire HRAP is inversely proportional to the performance $\eta$ of the agitation device. The global energetic performance of the selected agitator, according to the manufacturer, is 78%. On the other hand, the volumetric performance of the agitator measured as the ratio between the real effective flow rate ($m^3/s$) at the HRAP and the flow rate propelled by the propeller has been obtained from the fluid-dynamic simulation and is equivalent to 0.225/0.230 = 0.976.

This elevated volumetric performance was obtained by means of an optimization process of the geometry of the flow acceleration system until the parasite backflow currents were minimized, finally obtaining a space of only 10 cm around the propeller. In other words, in the proposed design, the real effective flow rate at the HRAP presented values of 97.6% of the flow rate propelled by the propeller, with a hydraulic loss of only 2.4%. This is due to the fact that the proposed design leads the flow towards the interior of a circular conduit, with small spaces between the propeller and the tube (10 mm). This way, the total performance of the flow accelerator is the following:

$$\textit{Total performance of the flow accelerator system = global performance of the}$$
$$\textit{agitator x volumetric performance = 0.78x0.976 = 0.75}$$

The paddlewheel values have been obtained as an average of the bibliographic values referenced above.

**Table 4. Agitation Performances**

| AGITATION PERFORMANCES | Design of the invention | Paddlewheel |
|---|---|---|
| Hydraulic + mechanical performance | 0.78 | 0.235 |
| Volumetric performance | 0.976 | 0.85 |
| Global performance | 0.75 | 0.2 |

With these performances and the load losses h of Table 3, we were able to calculate the power consumed by both systems:

**Table 5. Power consumed in both systems**

| 500 $m^2$ Pond | Power (W) | Power ($w/m^2$) |
|---|---|---|
| Conventional design | 434.13 | 0.868265 |
| Proposed design | 47.95 | 0.095902 |
| TOTAL ENERGY SAVINGS | 89% | 89% |

Table 5 shows how the savings in energy consumption with the proposed design were 89% with respect to the conventional system. These savings take into account the improvement of the hydraulic design (Table 3) and the improvement of the agitation device.

Example 2. Application of the HRAP reactor object of the present invention and described in Example 1 in a wastewater treatment plant with 4,500 $m^2$ HRAP reactors

The present example proposed the energetic advantage represented by the present invention when applied to a wastewater treatment plant made with HRAP's with respect to the conventional system with activated sludge. The treatment plant of the present example was designed with HRAP reactors with a unit area of 4,500 $m^2$, which is the usual size for large facilities.

The fluid-dynamic simulation by means of CFD techniques was carried out with the *ANSY fluent* program of a 4,500 $m^2$-HRAP according to the design of the invention, until the optimization of the parameters. The construction parameters ended up being the following:

H1 = 0.3 m
B1 =9m
B2=3m
H2 = 0.9 m

R = 4.5 m
L2 = 16.4 m
L1 = 24 m
L3 = 193x24x2x6x2 = 253 m
L4 = 0.6
L5 = 5 m
L6 = 4 m
L7 = 5 m
L8 = 1.8 m
H3 = 100 mm
D = 1,800 mm
D1 = 1,820 mm

The agitator selected is an ABS SB1800 model, with a diameter of 1,800 mm and low output speed (38 rpm), but with a high global performance = 0.8, according to the data provided by the manufacturer.

The other design parameters are those contained in Table 6.

**Table 6. Design parameters of the 4,500 m$^2$-HRAP reactor**

| STARTING DATA | New design | Conventional |
|---|---|---|
| Speed (m/s) | 0.256 | 0.256 |
| Density (kg/m$^3$) | 998.4 | 998.4 |
| $L_{contractions\ and\ expan}$ L1 (m) | 24 | |
| Roughness (mm) | 0.003 | 0.003 |
| Area (m$^2$) | 4,500 | 4,500 |

Based on the previous design parameters, the fluid-dynamic simulation allows obtaining the load losses h along the system as presented in Figure 10. Based on the load losses h calculated with the mathematical simulation, the localized loss coefficient K can be obtained according to the following formula:

$$h = K \times \frac{v^2}{2g}$$

These values are presented in Table 2, which was completed with the values of the bibliographic loss coefficients referenced above for the conventional system. For the purposes of comparison, the load loss values of the equivalent straight channel with a length L1 = 24 m were calculated, which resulted in a value equivalent to $K_{contraction\ region}$= $K_{expansion\ region}$=0.552. This value is 55% higher than the average K value of the expansions and contractions of the new design due to the larger hydraulic radius. The foregoing supposes an additional hydraulic improvement in the proposed system.

**Table 7. Load losses Coefficients**

| Loss coefficients | New design | Conventional |
|---|---|---|
| $K_{curves}$ | 0.31 | 2 |
| $K_{expansion\ region}$ (L = 24m) | 0.28 | 0.552 |
| $K_{contraction\ region}$ (L = 24m) | 0.21 | 0.552 |
| $K_{carbonation\ pit}$ | | 4 |

Based on the previous K values, it is possible to estimate the hydraulic improvements represented by the new configuration of the reactor, estimated as the reduction of the load loss in the entire reactor. The load losses for the conventional system were calculated by applying the Darcy equation after calculating the friction factor by means of the Colebrook-White equation, resolved using the Newton method up to a tolerance of 1e-16. We have been able to verify that this system provides the same values than the fluid-dynamic simulation. The results are presented in Table 8.

**Table 8. Load losses and Hydraulic Improvements of the design of the invention**

| 4,500 m² Pond | $h_{straight+accessories}$ (m) | $h_{curves}$ (m) | $h_{total}$ (m) |
|---|---|---|---|
| Conventional design | 0.050390 | 0.013370 | 0.063760 |
| Design of the invention | 0.032930 | 0.002080 | 0.035010 |
| HYDRAULIC IMPROVEMENTS | | | 45% |

According to Table 8, and given that the consumption of energy P (w) is proportional to the load loss h (m) according to the formula:

$$P = \frac{\rho \times Q \times g \times h}{\eta}$$

we were able to deduce that the new design represented a reduction in the consumption of energy due to hydraulic improvements of 45%.

However, the present invention also represents a reduction of energy due to the agitation system. The consumption of energy P in the entire HRAP is inversely proportional to the performance η of the agitation device.

The global energetic performance of the selected agitator, according to the manufacturer, is 80%. On the other hand, the volumetric performance of the agitator, measured as the ratio between the real effective flow rate (m³/s) at the HRAP and the flow rate propelled by the propeller, was obtained from the fluid-dynamic simulation and is equivalent to 0.691/0.743 = 0.93.

This elevated volumetric performance was obtained by means of an optimization process of the geometry of the flow acceleration system until the parasite backflow currents were minimized, finally obtaining a space of only 10 cm around the propeller. In other words, in the proposed design, the real effective flow rate at the HRAP presented values of 93% of the flow rate propelled by the propeller, with a hydraulic loss of only 7%. This is due to the fact that the proposed design leads the flow towards the interior of a circular conduit, with small spaces between the propeller and the tube (10 mm). This way, the total performance of the flow accelerator is the following:

*Total performance of the flow accelerator system = global performance of the agitator x volumetric performance = 0.8x0.93 = 0.74*

The paddlewheel values have been obtained as an average of the bibliographic values referenced above.

**Table 9. Agitation Performances**

| AGITATION PERFORMANCES | Design of the invention | Paddlewheel |
|---|---|---|
| Hydraulic + mechanical performance | 0.8 | 0.235 |
| Volumetric performance | 0.93 | 0.85 |
| Global performance | 0.74 | 0.2 |

With these performances and the load losses h of Table 8, we were able to calculate the power consumed by both systems:

**Table 10. Power consumed in both systems**

| 4,500 m² Pond | Power (W) | Power (w/m²) |
|---|---|---|
| Conventional design | 2,158.22 | 0.479604 |
| Design of the invention | 318.56 | 0.070792 |
| TOTAL ENERGY SAVINGS | 85% | 85% |

Table 5 shows how the savings in energy consumption with the proposed design were 85% with respect to the conventional system. These savings take into account the improvement of the hydraulic design (Table 3) and the improvement of the agitation device. Therefore, with the design of the invention a value of 0.07 w/m$^2$ was obtained, with respect to 0.479 w/m$^2$ of the conventional HRAP system.

To verify the importance of this energetic improvement, the energy consumption data of three types of municipal wastewater treatment plants (DQO = 700 mg/l, NT = 40 mg/l, PT = 10 mg/l) were compared. First, a treatment plant with the conventional activated sludge system with the elimination of N and P; second, a treatment plant with microalgae with conventional HRAP systems; and lastly, the treatment plant built with the improvements of the present invention. For practical purposes, we supposed that the pre-treatment and biomass separation and dehydration system was the same for the three plants, due to which we only compared the biological treatment reactor.

4,800 m$^3$/day treatment plant, made with 4,500 m$^2$ ponds: estimated effective surface: 8.1 ha = 81,000 m$^2$. Number of 4,500 m$^2$ ponds = 18 units.

**Table 11. Power consumed in the three wastewater treatment plants being compared**

| | Occupied surface (m$^2$) | Power consumed HRAP (w/m$^2$) | Power consumed (Kwh/day) | Power consumed at the biological reactor (kwh/m$^3$) | Energy savings |
|---|---|---|---|---|---|
| Activated sludge | 5,000 | | 1,420 | 0.30 | - |
| HRAP with paddlewheel | 81,000 | 0.479 | 932 | 0.19 | 37% |
| HRAP of the invention | 81,000 | 0.07 | 138 | 0.03 | 90% |

Table 11 shows how the HRAP with a conventional paddlewheel agitation system reduced the energy consumed by a conventional treatment plant with activated sludge in approximately 37%. This reduction can be insufficient in many cases to opt for this alternative treatment system, taking into account the disadvantage of the occupied surface.

However, with the system proposed by the present invention, energy consumption was reduced in 90% with respect to a conventional treatment plant, which is a very attractive value for the selection of this wastewater treatment technology due to its cost-effectiveness and performance.

**Claims**

1. A high rate algae pond (HRAP) reactor for the cultivation of microalgae or mixotrophic microalgae-bacterial cultures comprising:

   - a flow channel, through which a sheet of water with a water level ranging between 0.2 and 0.6 m passes, in the shape of a rounded rectangle with four sections: two straight parallel segments, separated by an intermediate partition and joined at their ends with 180° semicircular curves, each one of them provided with an entry and an exit depending on the direction in which the water sheet runs; and
   - at least a first agitation system for said water sheet, located between the exit of one of the curves and the end of the contiguous straight segment of the channel,

   wherein at least the first curve, contiguous to the agitation system, is 2 to 4 times narrower than the main straight segments of the channel and has an average curvature radius 1 to 2 times larger than the width of the curve, the entry of said curve being joined to the contiguous straight segment by means of a first transition region consisting of a narrowing from the straight segment to the entry of the curve in the shape of an isosceles trapezoid, with flat walls, presenting a depth increase in the same proportion as the narrowing of its width with respect to the straight segment, and a length such that the ratio between the length and the width of the straight segment of the channel is comprised between 1 and 3;
   the reactor being **characterized in that**:

   - the agitation system is a flow accelerator that comprises a flow acceleration device with an axial flow propeller, producing an axial flow at rotational speed equal to or lower than 100 rpm, which is housed inside a closed tubular walled conduit with two open ends for the passage of the entire water sheet through its interior, the highest point of which is at a height below the lower level of water at the inlet of the agitation system, and with a distance between the inner diameter of the conduit and the end of the propellers comprised between 2 and 20 mm; said agitation system being located between two transition sections, the first one joining the rectangular section of the curve with the circular section of the conduit at one end, and the second one joining the circular section of the conduit with the section of a second transition region of the channel at the other end, with the same configuration as the first, that is also joined to the contiguous straight segment;

the cross sectional area of all the sections of the reactor being constant, and the flow speed being also constant between 0.2 and 0.4 m/s.

2. The reactor according to claim 1, wherein the width of the straight segments of the channel is comprised between 0.5 and 25 m.

3. The reactor according to any one of the previous claims, wherein the second curve presents the same configuration as the first, and both the entry and exit of said second curve are joined to the ends of the contiguous straight segments by means of a third and fourth transition regions, like the one defined as the entry of the first curve, such that the reactor comprises: the first straight segment; the first transition region up to the first curve; the first curve; the agitation system; the second transition region up to the second straight segment; the second straight segment; the third transition region up to the second curve; the second curve; and the fourth transition region up to the first straight segment of the channel.

4. The reactor according to any one of the claims 1 or 2, wherein the second curve presents the same configuration as the first, the entry of said second curve being joined to the end of the contiguous straight section by means of a third transition region as the first one, and the exit of said second curve being connected to one of the ends of a second agitation system as the first one, connected in turn in its opposite end to a fourth transition region towards the contiguous straight segment, such that the reactor comprises: the first straight segment; the first transition region up to the first curve; the first curve; the first agitation system; the second transition region up to the second straight segment; the second straight segment; the third transition region up to the second curve; the second curve; the second agitation system; and the fourth transition region up to the first straight segment of the channel.

5. The reactor according to any one of the previous claims, wherein at least one of the two curves comprises a deflector with the same curvature radius than the curve itself.

6. The reactor according to any one of the previous claims, wherein the length of the closed tubular walled conduit of the agitation system is comprised between 1 m and 5 m, and the highest point of said conduit is located at a depth below the level of the water sheet at the inlet of the agitation system comprised between 100 and 400 mm, including both limits.

7. The reactor according to any one of the previous claims, wherein the rotational speed of the flow acceleration device is comprised between 30 and 80 rpm, including both limits.

8. The reactor according to any one of the previous claims, wherein the agitation system comprises a solids settling pit housed at the inlet of said system and before the inlet of the closed tubular walled conduit, said solids settling pit presenting a rectangular shape in the upper portion with a length between 0.5 and 1 m, and a width equal to the width of the curve, and a horizontal flat bottom with a length equal to the upper region and a width between 0.4 and 0.6 m in the lower portion, matching the lower portion of the closed tubular walled conduit, the upper and lower portions being joined by inclined planes.

9. The reactor according to the previous claim, wherein the first transition section between the solids settling pit and the inlet of the closed tubular walled conduit wherein the flow acceleration device is housed consists of a closed conduit presenting a rectangular end with a width and height corresponding to the shape of the contiguous curve to which is connected, and a second circular end with a diameter equal to the diameter of the closed tubular walled conduit to which it is connected and wherein the flow acceleration device is located, with downslopes comprised between 1:4 and 1:6.

10. The reactor according to the previous claim, wherein the second transition section located between the outlet of the closed tubular walled conduit and the transition region of the channel that is connected to the outlet of the agitation system towards the straight segment of the channel is equal to the first one.

**11.** The reactor according to claim 10, wherein the agitation system comprises a carbonation system between the second transition section located at the outlet of the closed tubular walled conduit and the transition region of the channel located at the outlet of the agitation system and that connects said system to the contiguous straight segment of the channel.

**12.** The reactor according to the previous claim, wherein the carbonation system consists of a pit without a deflector partition, located at the bottom of the agitation system and having a width equivalent to that of the flow channel and a length comprised between half and the double of the width of the channel, and that it further comprises at least one $CO_2$ injection device inside the pit, located at the base thereof and next to the wall opposite to the incoming direction of the sheet of water, with a distance from the upper face of the device through which the $CO_2$ is injected to the bottom of the flow channel comprised between 0.5 and 1.5 m, including both limits.

**13.** The reactor according to claim 1, consisting of:

a) two straight segments with a width between 0.5 and 25 m;
b) two curves at the ends of the straight segments, with an average curvature radius:channel width ratio comprised between 1 and 2, and a deflector with the same curvature radius than the curve;
c) four transition regions of the channel, two between the ends of the straight segments of the channel joined to the entries of the contiguous curves and two between the agitation systems and the contiguous straight segments to which they are joined; where the ratio between the straight segment width and the curve width is comprised between 2 and 4, such that the depth is gradually increased in the same value along the length of the transition region, the ratio between the transition region length and the width of the straight segment of the channel being comprised between 1 and 3;
d) two agitation systems, located at the exit of the curves, containing the flow acceleration device located inside the closed tubular walled conduit with two open ends for the passage of the water sheet, and the upper portion of which is at a height below the level of the water sheet at the inlet of the agitation system, said agitation systems further comprising:

- a solids settling pit, with a length comprised between 0.5 and 1 m and with a horizontal flat bottom in the lower portion with a width comprised between 40 and 60 cm, joined to the upper portion by inclined planes, wherein the horizontal flat bottom matches the lower portion of the closed tubular walled conduit, housing a submersible pump for the extraction of settled solids;
- a first transition section between the solids settling pit and the closed tubular walled conduit, consisting of a hopper made with metalwork techniques for the transition between the rectangular shape of the curve and the circular shape of the closed tubular walled conduit, formed by four triangles and four curved segments;
- the closed tubular walled conduit, which is a circular tube wherein the flow accelerator is concentrically located, with a length comprised between 1 m and 5 m;
- a second transition section, with the same configuration as the first one, between the outlet of the closed tubular walled conduit and a carbonation system; and
- the carbonation system, consisting of a pit without deflector partitions, located at the bottom of the agitation system and with a width equivalent to the width of the flow channel and a length comprised between half and the double of the width of the channel, and that it further comprises at least one $CO_2$ injection device inside the pit, located at the base thereof and next to the wall opposite to the incoming direction of the water sheet, with a distance from the upper face of the device through which the $CO_2$ is injected to the bottom of the flow channel comprised between 0.5 and 1.5 m, including both limits.

**Patentansprüche**

**1.** HRAP (High Rate Algae Pond)-Reaktor für die Züchtung von Mikroalgen oder mixotrophen Mikroalgen-Bakterien-Kulturen, umfassend:

- einen Flusskanal, durch den eine Wasserschicht mit einem Wasserpegel zwischen 0,2 und 0,6 m hindurchgeht und der die Form eines gerundeten Rechtecks mit vier Abschnitten aufweist, nämlich zwei parallelen Segmenten, die durch eine dazwischenliegende Trennwand getrennt werden und deren Enden durch 180°-halbkreisförmige Kurven verbunden werden, die jeweils mit einem Eingang und einem Ausgang in Entsprechung zu der Richtung, in der die Wasserschicht läuft, versehen sind, und
- wenigstens ein erstes Bewegungssystem für die Wasserschicht, das zwischen dem Ausgang einer der Kurven und dem Ende des anschließenden geraden Segments des Kanals angeordnet ist,

wobei wenigstens die erste Kurve, die an das Bewegungssystem anschließt, 2 bis 4 mal schmäler als die geraden Hauptsegmente des Kanals ist und einen durchschnittlichen Krümmungsradius aufweist, der 1 bis 2 mal größer als die Breite der Kurve ist, wobei der Eingang der Kurve mit dem anschließenden geraden Segment über einen ersten Übergangsbereich verbunden ist, der aus einer Verschmälerung von dem geraden Segment zu dem Eingang der Kurve in der Form eines gleichschenkligen Trapezoids besteht, flache Wände aufweist, die eine Tiefenvergrößerung proportional zu der Verschmälerung der Breite in Bezug auf das gerade Segment aufweisen, und eine derartige Länge aufweist, dass das Verhältnis zwischen der Länge und der Breite des geraden Segments des Kanals zwischen 1 und 3 beträgt,
wobei der Reaktor **dadurch gekennzeichnet ist, dass**:

- das Bewegungssystem ein Flussbeschleuniger ist, der eine Flussbeschleunigungseinrichtung mit einem Axialflusspropeller umfasst, der einen Axialfluss mit einer Drehgeschwindigkeit gleich oder kleiner 100 U/min erzeugt, in einer rohrförmigen Leitung mit einer geschlossenen Wand und mit zwei offenen Enden für den Durchgang der gesamten Wasserschicht durch ihr Inneres aufgenommen ist, deren höchster Punkt eine Höhe unter dem unteren Wasserpegel am Einlass des Bewegungssystems ist, wobei die Distanz zwischen dem Innendurchmesser der Leitung und dem Ende der Propeller zwischen 2 und 20 mm beträgt, wobei das Bewegungssystem zwischen zwei Übergangsabschnitten angeordnet ist, von denen der erste den rechteckigen Abschnitt der Kurve mit dem kreisrunden Abschnitt der Leitung an einem Ende verbindet und der zweite den kreisrunden Abschnitt der Leitung mit dem Abschnitt eines zweiten Übergangsbereichs des Kanals an dem anderen Ende verbindet, die gleiche Konfiguration wie der erste aufweist und mit dem anschließenden geraden Segment verbunden ist,
- die Querschnittfläche aller Abschnitte des Reaktors konstant ist und auch die Flussgeschwindigkeit konstant zwischen 0,2, und 0,4 m/s beträgt.

2. Reaktor nach Anspruch 1, wobei die Breite der geraden Segmente des Kanals zwischen 0,5 und 25 m beträgt.

3. Reaktor nach einem der vorstehenden Ansprüche, wobei die zweite Kurve die gleiche Konfiguration aufweist wie die erste und der Eingang und der Ausgang der zweiten Kurve mit den Enden des anschließenden geraden Segmente über dritte und vierte Übergangsbereiche verbunden sind, die dem als dem Eingang der ersten Kurve definierten ähnlich sind, sodass der Reaktor das erste gerade Segment, den ersten Übergangsbereich bis zu der ersten Kurve, die erste Kurve, das Bewegungssystem, den zweiten Übergangsbereich bis zu dem zweiten geraden Segment, das zweite gerade Segment, den dritten Übergangsbereich bis zu der zweiten Kurve, die zweite Kurve, und den vierten Übergangsbereich bis zu dem ersten geraden Segment des Kanals umfasst.

4. Reaktor nach einem der Ansprüche 1 oder 2, wobei die zweite Kurve die gleiche Konfiguration aufweist wie die erste, der Eingang der zweiten Kurve mit dem Ende des anschließenden geraden Abschnitts über einen dritten Übergangsbereich verbunden ist wie die erste und der Ausgang der zweiten Kurve mit einem der Enden eines zweiten Bewegungssystems verbunden ist wie die erste und wiederum an ihrem gegenüberliegenden Ende mit einem vierten Übergangsbereich zu dem anschließenden geraden Segment verbunden ist, sodass der Reaktor das erste gerade Segment, den ersten Übergangsbereich bis zu der ersten Kurve, die erste Kurve, das erste Bewegungssystem, den zweiten Übergangsbereich bis zu dem zweiten geraden Segment, das zweite gerade Segment, den dritten Übergangsbereich bis zu der zweiten Kurve, die zweite Kurve, das zweite Bewegungssystem und den vierten Übergangsbereich bis zu dem ersten geraden Segment des Kanals umfasst.

5. Reaktor nach einem der vorstehenden Ansprüche, wobei wenigstens eine der zwei Kurven ein Ablenkglied mit dem gleichen Krümmungsradius wie die Kurve selbst umfasst.

6. Reaktor nach einem der vorstehenden Ansprüche, wobei die Länge der rohrförmigen Leitung mit einer geschlossenen Wand des Bewegungssystems zwischen 1 und 5 m beträgt und der höchste Punkt der Leitung mit einer Tiefe unter dem Pegel der Wasserschicht an dem Einlass des Bewegungssystems zwischen 100 und 400 mm einschließlich der beiden Grenzwerte angeordnet ist.

7. Reaktor nach einem der vorstehenden Ansprüche, wobei die Drehgeschwindigkeit der Flussbeschleunigungseinrichtung zwischen 30 und 80 U/min einschließlich der beiden Grenzwerte beträgt.

8. Reaktor nach einem der vorstehenden Ansprüche, wobei das Bewegungssystem ein Feststoff-Absatzbecken umfasst, das an dem Einlass des Systems und vor dem Einlass der rohrförmigen Leitung mit einer geschlossenen Wand aufgenommen ist, wobei das Feststoff-Absatzbecken eine rechteckige Form im oberen Teil mit einer Länge zwischen 0,5 und 1 m und einer Breite gleich der Breite der Kurve und einen horizontalen, flachen Boden mit einer Länge gleich derjenigen des oberen Bereichs und einer Breite zwischen 0,4 und 0,6 in dem unteren Teil in Entsprechung zu dem unteren Teil der rohrförmigen Leitung mit einer geschlossenen Wand aufweist, wobei die oberen und unteren Teile durch geneigte Ebenen miteinander verbunden sind.

9. Reaktor nach dem vorstehenden Anspruch, wobei der erste Übergangsabschnitt zwischen dem Feststoff-Absatzbecken und dem Einlass der rohrförmigen Leitung mit einer geschlossenen Wand, in der die Flussbeschleunigungseinrichtung aufgenommen ist, aus einer geschlossenen Leitung besteht, die ein rechteckiges Ende mit einer Breite und Höhe in Entsprechung zu der Form der anschließenden Kurve, mit der er verbunden ist, und ein zweites kreisrundes Ende mit einem Durchmesser gleich dem Durchmesser der rohrförmigen Leitung mit einer geschlossenen Wand, mit der er verbunden ist und in der die Flussbeschleunigungseinrichtung angeordnet ist, mit Gefällen zwischen 1:4 und 1:6 aufweist.

10. Reaktor nach dem vorstehenden Anspruch, wobei der zweite Übergangsabschnitt, der zwischen dem Auslass der rohrförmigen Leitung mit einer geschlossenen Wand und dem Übergangsbereich des Kanals, der mit dem Auslass des Bewegungssystems zu dem geraden Segment des Kanals verbunden ist, angeordnet ist, gleich dem ersten ist.

11. Reaktor nach Anspruch 10, wobei das Bewegungssystem ein Karbonisierungssystem zwischen dem zweiten Übergangsabschnitt, der an dem Auslass der rohrförmigen Leitung mit einer geschlossenen Wand angeordnet ist, und dem Übergangsbereich des Kanals, der an dem Auslass des Bewegungssystems angeordnet ist und das System mit dem anschließenden geraden Segment des Kanals verbindet, umfasst.

12. Reaktor nach dem vorstehenden Anspruch, wobei das Karbonisierungssystem aus einem Becken ohne eine Ablenkungsglied-Trennwand besteht, das an dem Boden des Bewegungssystems angeordnet ist und eine Breite in Entsprechung zu derjenigen des Flusskanals und eine Länge zwischen der halben und der doppelten Breite des Kanals aufweist, und weiterhin wenigstens eine $CO_2$-Einspritzeinrichtung in dem Becken umfasst, die an der Basis desselben und neben der Wand gegenüber der Eingangsrichtung der Wasserschicht mit einer Distanz von der oberen Fläche der Einrichtung, durch die das $CO_2$ eingespritzt wird, zu dem Boden des Flusskanals zwischen 0,5 und 1,5 m einschließlich der beiden Grenzwerte angeordnet ist.

13. Reaktor nach Anspruch 1, der besteht aus:

a) zwei geraden Segmenten mit einer Breite zwischen 0,5 und 25 m,
b) zwei Kurven an den Enden der geraden Segmente mit einem durchschnittlichen Krümmungsradius:Kanalbreite-Verhältnis zwischen 1 und 2 und einem Ablenkglied mit dem gleichen Krümmungsradius wie die Kurve,
c) vier Übergangsbereichen des Kanals, von denen zwei zwischen den Enden der geraden Segmente des Kanals angeordnet sind und mit den Eingängen der anschließenden Kurven verbunden sind und zwei zwischen den Bewegungssystemen und den anschließenden geraden Segmenten, mit denen sie verbunden sind, angeordnet sind, wobei das Verhältnis zwischen der Breite des geraden Segments und der Breite der Kurve zwischen 2 und 4 beträgt, sodass die Tiefe allmählich mit dem gleichen Wert entlang der Länge des Übergangsbereichs größer wird, wobei das Verhältnis zwischen der Länge des Übergangsbereichs und der Breite des geraden Segments des Kanals zwischen 1 und 3 beträgt,
d) zwei Bewegungssystemen, die an dem Ausgang der Kurven angeordnet sind und die Flussbeschleunigungseinrichtung enthalten, die in der rohrförmigen Leitung mit einer geschlossenen Wand und den zwei offenen Enden für den Durchgang der Wasserschicht angeordnet ist, deren oberer Teil auf einer Höhe unter dem Pegel der Wasserschicht an dem Einlass des Bewegungssystems liegt, wobei die Bewegungssysteme weiterhin umfassen:

- ein Feststoff-Absatzbecken mit einer Länge zwischen 0,5 und 1 m und einem horizontalen flachen Boden in dem unteren Teil mit einer Breite zwischen 40 und 60 cm, der mit dem oberen Teil über geneigte Ebenen verbunden ist, wobei der horizontale, flache Boden dem unteren Teil der rohrförmigen Leitung mit einer geschlossenen Wand entspricht und eine eintauchbare Pumpe für die Beseitigung von abgesetzten Feststoffen enthält,

- einen ersten Übergangsabschnitt zwischen dem Feststoff-Absetzbecken und der rohrförmigen Leitung mit einer geschlossenen Wand, der aus einem mit Metallverarbeitungstechniken ausgebildeten Trichter für den Übergang zwischen der rechteckigen Form der Kurve und der kreisrunden Form der rohrförmigen Leitung mit einer geschlossenen Wand besteht und durch vier Dreiecke und vier kurvenförmige Segmente gebildet wird,
- die rohrförmige Leitung mit einer geschlossenen Wand, die ein kreisrundes Rohr ist, in dem der Flussbeschleuniger konzentrisch angeordnet ist, und eine Länge zwischen 1 m und 5 m aufweist,
- einen zweiten Übergangsabschnitt mit der gleichen Konfiguration wie der erste, der zwischen dem Auslass der rohrförmigen Leitung mit einer geschlossenen Wand und einem Karbonisierungssystem angeordnet ist, und
- das Karbonisierungssystem, das aus einem Becken ohne Ablenkungsglied-Trennwänden besteht, an dem Boden des Bewegungssystems angeordnet ist und eine Breite in Entsprechung zu der Breite des Flusskanals und eine Länge zwischen der halben und der doppelten Breite des Kanals aufweist, wobei es weiterhin wenigstens eine $CO_2$-Einspritzeinrichtung in dem Becken aufweist, das an seiner Basis und neben der Wand gegenüber der Einspritzrichtung der Wasserschicht angeordnet ist, wobei die Distanz von der oberen Fläche der Einrichtung, durch die das $CO_2$ eingespritzt wird, und dem Boden des Flusskanals zwischen 0,5 und 1,5 m einschließlich der beiden Grenzwerte beträgt.

**Revendications**

1. Réacteur ouvert d'algues à haut débit (HRAP) pour la culture des micro-algues ou les cultures mixotrophes de micro-algues - bactéries comprenant :

   - un canal d'écoulement, à travers lequel passe une lame d'eau avec un niveau d'eau compris entre 0,2 et 0,6 m, sous la forme d'un rectangle arrondi avec quatre sections : deux segments parallèles droits, séparés par une séparation intermédiaire et assemblés au niveau de leurs extrémités avec des courbes semi-circulaires à 180°, chacun d'entre eux étant prévu avec une entrée et une sortie en fonction de la direction dans laquelle la lame d'eau circule ; et
   - au moins un premier système d'agitation pour ladite lame d'eau, situé entre la sortie de l'une des courbes et l'extrémité du segment droit contigu du canal,

   dans lequel au moins la première courbe, contiguë par rapport au système d'agitation, est 2 à 4 fois plus étroite que les segments droits principaux du canal et a un rayon de courbure moyen 1 à 2 fois plus grand que la largeur de la courbe, l'entrée de ladite courbe étant assemblée au segment droit contigu au moyen d'une première région de transition se composant d'un rétrécissement du segment droit à l'entrée de la courbe se présentant sous la forme d'un trapèze isocèle, avec des parois plates, présentant une augmentation de profondeur dans la même proportion que le rétrécissement de sa largeur par rapport au segment droit et à une longueur de sorte que le rapport entre la longueur et la largeur du segment droit du canal est compris entre 1 et 3 ;
   le réacteur étant **caractérisé en ce que** :

   - le système d'agitation est un accélérateur de flux qui comprend un dispositif d'accélération de flux avec une hélice axiale, produisant un flux axial à une vitesse de rotation égale ou inférieure à 100 rpm (tours par minute) qui est logée à l'intérieur d'un conduit à paroi tubulaire fermé avec deux extrémités ouvertes pour le passage de toute la lame d'eau dans son intérieur, dont le point le plus haut est à une hauteur au-dessous du niveau inférieur d'eau à l'entrée du système d'agitation, et avec un distance entre le diamètre interne du conduit et l'extrémité des hélices comprise entre 2 et 20 mm ; ledit système d'agitation étant positionné entre deux sections de transition, la première assemblant la section rectangulaire de la courbe avec la section circulaire du conduit au niveau d'une extrémité, et la seconde assemblant la section circulaire du conduit avec la section d'une seconde région de transition du canal au niveau de l'autre extrémité, avec la même configuration que la première, qui est également assemblée au segment droit contigu ;

   la surface transversale de toutes les sections du réacteur étant constante, et la vitesse d'écoulement étant également constante comprise entre 0,2 et 0,4 m/s.

2. Réacteur selon la revendication 1, dans lequel la largeur des segments droits du canal est comprise entre 0,5 et 25 m.

3. Réacteur selon l'une quelconque des revendications précédentes, dans lequel la seconde courbe présente la même configuration que la première, et à la fois l'entrée et la sortie de ladite seconde courbe sont assemblées aux extrémités des segments droits contigus au moyen d'une troisième et d'une quatrième région de transition, comme celle définie en tant qu'entrée de la première courbe, de sorte que le réacteur comprend : un premier segment droit ; la première région de transition jusqu'à la première courbe ; la première courbe ; le système d'agitation ; la deuxième région de transition jusqu'au second segment droit ; le second segment droit ; la troisième région de transition jusqu'à la seconde courbe ; la seconde courbe ; et la quatrième région de transition jusqu'au premier segment droit du canal.

4. Réacteur selon l'une quelconque des revendications 1 ou 2, dans lequel la seconde courbe présente la même configuration que la première, l'entrée de ladite seconde courbe étant assemblée à l'extrémité de la section droite contiguë au moyen d'une troisième région de transition comme la première, et la sortie de ladite seconde courbe étant raccordée à l'une des extrémités d'un second système d'agitation comme le premier, raccordée à son tour dans son extrémité opposée à une quatrième région de transition vers le segment droit contigu, de sorte que le réacteur comprend : le premier segment droit ; la première région de transition jusqu'à la première courbe ; la première courbe ; le premier système d'agitation ; la deuxième région de transition jusqu'au second segment droit ; le second segment droit ; la troisième région de transition jusqu'à la seconde courbe ; la seconde courbe ; le second système d'agitation ; et la quatrième région de transition jusqu'au premier segment droit du canal.

5. Réacteur selon l'une quelconque des revendications précédentes, dans lequel au moins l'une des deux courbes comprend un déflecteur avec le même rayon de courbure que la courbure elle-même.

6. Réacteur selon l'une quelconque des revendications précédentes, dans lequel la longueur du conduit à paroi tubulaire fermé du système d'agitation est comprise entre 1 m et 5 m, et le point le plus haut dudit conduit est situé à une profondeur au-dessous du niveau de la lame d'eau à l'entrée du système d'agitation compris entre 100 et 400 mm, comprenant les deux limites.

7. Réacteur selon l'une quelconque des revendications précédentes, dans lequel la vitesse de rotation du dispositif d'accélération de flux est comprise entre 30 et 80 rpm, comprenant les deux limites.

8. Réacteur selon l'une quelconque des revendications précédentes, dans lequel le système d'agitation comprend un bac de décantation de solides logé à l'entrée dudit système, et avant l'entrée du conduit à paroi tubulaire fermé, ledit bac de décantation de solides présentant une forme rectangulaire dans la partie supérieure, avec une longueur comprise entre 0,5 et 1 m, et une largeur égale à la largeur de la courbe, et un fond plat horizontal avec une longueur égale à la région supérieure et une largeur comprise entre 0,4 et 0,6 m dans la partie inférieure, correspondant à la partie inférieure du conduit à paroi tubulaire fermé, les parties supérieure et inférieure étant assemblées par des plans inclinés.

9. Réacteur selon la revendication précédente, dans lequel la première section de transition entre le bac de décantation de solides et l'entrée du conduit à paroi tubulaire fermé où le dispositif d'accélération de flux est logé, se compose d'un conduit fermé présentant une extrémité rectangulaire avec une largeur et une hauteur correspondant à la forme de la courbe contiguë à laquelle il est raccordé, et une seconde extrémité circulaire avec un diamètre égal au diamètre du conduit à paroi tubulaire fermé auquel il est raccordé, et où le dispositif d'accélération de flux est positionné, avec des pentes descendantes comprises entre 1:4 et 1:6.

10. Réacteur selon la revendication précédente, dans lequel la seconde section de transition positionnée entre la sortie du conduit à paroi tubulaire fermé et la région de transition du canal qui est raccordée à la sortie du système d'agitation vers le segment droit du canal est égale à la première.

11. Réacteur selon la revendication 10, dans lequel le système d'agitation comprend un système de carbonatation entre la seconde section de transition située à la sortie du conduit à paroi tubulaire fermé et la région de transition du canal située à la sortie du système d'agitation et qui raccorde ledit système audit segment droit contigu du canal.

12. Réacteur selon la revendication précédente, dans lequel le système de carbonatation se compose d'un bac sans séparation de déflecteur, situé au fond du système d'agitation et ayant une largeur équivalente à celle du canal d'écoulement et une longueur comprise entre la moitié et le double de la largeur du canal, et qui comprend en outre au moins un dispositif d'injection de $CO_2$ à l'intérieur du bac, situé au niveau de sa base et à côté de la paroi opposée à la direction entrante de la lame d'eau, avec une distance par rapport à la face supérieure du dispositif à travers laquelle le $CO_2$ est injecté au fond du canal d'écoulement comprise entre 0,5 et 1,5 m, comprenant les deux limites.

**13.** Réacteur selon la revendication 1, se composant de :

a) deux segments droits avec une largeur compris entre 0,5 et 25 m ;

b) deux courbes au niveau des extrémités des segments droits, avec un rapport de rayon de courbure moyen sur largeur de canal compris entre 1 et 2, et un déflecteur avec le même rayon de courbure que la courbe ;

c) quatre régions de transition du canal, deux entre les extrémités des segments droits du canal assemblés aux entrées des courbes contiguës et deux entre les systèmes d'agitation et les segments droits contigus auxquels elles sont assemblées ; où le rapport entre la largeur de segment droit et la largeur de courbe est compris entre 2 et 4, de sorte que la profondeur est progressivement augmentée selon la même valeur le long de la longueur de la région de transition, le rapport entre la longueur de région de transition et la largeur du segment droit du canal étant compris entre 1 et 3 ;

d) deux systèmes d'agitation positionnés à la sortie des courbes, contenant le dispositif d'accélération de flux situé à l'intérieur du conduit à paroi tubulaire fermé avec deux extrémités ouvertes pour le passage de la lame d'eau, dont la partie supérieure est à une hauteur au-dessous du niveau de la lame d'eau à l'entrée du système d'agitation, lesdits systèmes d'agitation comprenant en outre :

- un bac de décantation de solides, avec une longueur comprise entre 0,5 et 1 m et avec un fond plat horizontal dans la partie inférieure avec une largeur comprise entre 40 et 60 cm, assemblé à la partie supérieure par des plans inclinés, dans lequel le fond plat horizontal correspond à la partie inférieure du conduit à paroi tubulaire fermé, logeant une pompe submersible pour l'extraction des solides décantés ;

- une première section de transition entre le bac de décantation de solides et le conduit à paroi tubulaire fermé, se composant d'une trémie réalisée avec des techniques de métallerie pour la transition entre la forme rectangulaire de la courbe et la forme circulaire du conduit à paroi tubulaire fermé, formé par quatre triangles et quatre segments incurvés ;

- le conduit à paroi tubulaire fermé, qui est un tube circulaire où l'accélérateur de flux est positionné de manière concentrique, avec une longueur comprise entre 1 m et 5 m ;

- une seconde section de transition avec la même configuration que la première, entre la sortie du conduit à paroi tubulaire fermé et un système de carbonatation ; et

- le système de carbonatation, se composant d'un bac sans séparations de déflecteur, positionné au fond du système d'agitation et avec une largeur équivalente à la largeur du canal d'écoulement et une longueur comprise entre la moitié et le double de la largeur du canal, et de sorte qu'il comprend en outre au moins un dispositif d'injection de $CO_2$ à l'intérieur du bac, positionné au niveau de sa base et à proximité de la paroi opposée à la direction entrante de la lame d'eau, avec une distance par rapport à la face supérieure du dispositif à travers laquelle le $CO_2$ est injecté, jusqu'au fond du canal d'écoulement comprise entre 0,5 et 1,5 m, comprenant les deux limites.

**FIG. 1 (state of the art)**

**FIG. 2 (Liffman, Paterson et al., 2013; state of the art)**

**FIG. 3 (state of the art)**

FIG. 4

FIG. 5

FIG. 6

FIG. 7

FIG. 8

**FIG. 9**

**FIG. 10**

FIG. 11

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- ES P201231485
- US 3885370 A
- US 8142167 B
- US 3969249 A
- EP 2524962 A1
- MX 201201334 A
- US 3855370 A
- WO P201231485 A

### Non-patent literature cited in the description

- Culturing microalgae in outdoor ponds. **BOROWITZKA, M.** Algal Culturing Techniques. Elsevier Academic Press, 2005, 204-218
- **CHIARAMONTI, D. ; M. PRUSSI et al.** Review of energy balance in raceway ponds for microalgae cultivation: Re-thinking a traditional system is possible. *Applied Energy,* 2013, vol. 102 (0), 101-111
- **HAGER, W. H.** Wastewater Hydraulics, Theory and practice. 2010, 33
- **GARCÍA, J. ; Y MUJERIEGO, R.** Oxigenacion fotosintética en lagunas para la depuración de aguas residuales. *Tecnología del Agua,* 1999, vol. 195, 57-64
- **LIFFMAN, K. ; D. A. PATERSON et al.** Comparing the energy efficiency of different high rate algal raceway pond designs using computational fluid dynamics. *Chemical Engineering Research and Design,* 2013, vol. 91 (2), 221-226
- **LUNDQUIST TJ ; W. I. ; QUINN NWT ; BENEMANN JR.** A realistic technology and engineering assessment of algae biofuel production. *Energy Biosciences Institute,* 2010
- **RICHMOND, A.** Handbook of microalgal mass culture. Blackwell science Ltd, 2003
- **SOMPECH, K. ; Y. CHISTI et al.** Design of raceway ponds for producing microalgae. *Biofuels,* 2012, vol. 3 (4), 387-397
- **WEISSMAN JC ; T. D. ; GOEBEL RP.** Design and operation of an outdoor microalgae test facility, Final Subcontract Report. Microbial Products Inc, 1989